# FASCICULE DE BREVET EUROPEEN

(11) **EP 4 308 715 B1**
(45) Date de publication et mention de la délivrance du brevet: **13.05.2026**
(21) Numéro de dépôt: 22715347.5
(22) Date de dépôt: 18.03.2022
(51) Int. Cl.: C12P 13/10

(54) **MOLÉCULE HYBRIDE COMPRENANT UN FRAGMENT FC D'ANTICORPS ET AU MOINS UN PEPTIDE CITRULLINÉ DÉRIVÉ DE LA FIBRINE, ET SES UTILISATIONS**
HYBRIDMOLEKÜL MIT EINEM ANTIKÖRPER-FC-FRAGMENT UND MINDESTENS EINEM CITRULLINIERTEN PEPTID AUS FIBRIN SOWIE VERWENDUNGEN DAVON
HYBRID MOLECULE COMPRISING AN ANTIBODY FC FRAGMENT AND AT LEAST ONE FIBRIN-DERIVED CITRULLINATED PEPTIDE, AND USES THEREOF

(30) Priorité: 19.03.2021 FR 2102803
(43) Date de publication de la demande: 24.01.2024
(73) Titulaire: Université Paul Sabatier Toulouse III, 31400 Toulouse (FR); Institut National de la Santé et de la Recherche Médicale (INSERM), 75013 Paris (FR); Centre Hospitalier Universitaire de Toulouse, 31300 Toulouse (FR); Centre Hospitalier Universitaire de Montpellier, 34090 Montpellier (FR); UNIVERSITE DE MONTPELLIER, 34090 Montpellier (FR); Arthritis Recherche & Developpement, 92200 Neuilly sur Seine (FR)
(72) Inventeur: COMBES, Eve, 34560 VILLEVEYRAC (FR); ROBERT, Bruno, 34298 MONTPELLIER CEDEX 5 (FR); MARTINEAU, Pierre, 34090 MONTPELLIER (FR); LOUIS-PLENCE, Pascale, 34270 SAINT MATHIEU DE TREVIERS (FR); VILLALBA, Martin, 34070 MONTPELLIER (FR); JORGENSEN, Christian, 34980 SAINT-CLEMENT DE RIVIERE (FR); TROEGELER, Anthony, 31830 PLAISANCE DU TOUCH (FR); CLAVEL, Cyril, 31470 SAINT FOY DE PEYROLIERES (FR); SERRE, Guy, Bruno, René, 31100 TOULOUSE (FR)
(74) Mandataire: Plasseraud IP
(86) Numéro de dépôt international: PCT/FR2022/050504
(87) Numéro de publication internationale: WO 2022/195237

(56) Documents cités:
- EP-B1- 2 176 298
- US-A1- 2009 042 291
- ALETAHA DANIEL: "Precision medicine and management of rheumatoid arthritis", vol. 110, 1 June 2020 (2020-06-01), GB, pages 102405, XP055874103, ISSN: 0896-8411, Retrieved from the Internet <URL:https://www.sciencedirect.com/science/article/pii/S0896841120300068/pdfft?md5=20219f055e5f236f398cab5ef34dc309&pid=1-s2.0-S0896841120300068-main.pdf> DOI: 10.1016/j.jaut.2020.102405
- ANTONIO MANZO ET AL: "Secondary and ectopic lymphoid tissue responses in rheumatoid arthritis: from inflammation to autoimmunity and tissue damage/remodeling", IMMUNOLOGICAL REVIEWS, vol. 233, no. 1, 1 January 2010 (2010-01-01), US, pages 267 - 285, XP055218423, ISSN: 0105-2896, DOI: 10.1111/j.0105-2896.2009.00861.x
- WU CHAO-YI ET AL: "Anti-Citrullinated Protein Antibodies in Patients with Rheumatoid Arthritis: Biological Effects and Mechanisms of Immunopathogenesis", INTERNATIONAL JOURNAL OF MOLECULAR SCIENCES, vol. 21, no. 11, 1 January 2020 (2020-01-01), Basel, CH, pages 4015, XP055873913, ISSN: 1661-6596, DOI: 10.3390/ijms21114015
- DAMIANA TYRILLSHALL ET AL: "Citrullination of fibrinogen by peptidylarginine deiminase 2 impairs fibrin clot structure", CLINICA CHIMICA ACTA, ELSEVIER BV, AMSTERDAM, NL, vol. 501, 12 November 2019 (2019-11-12), pages 6 - 11, XP085994478, ISSN: 0009-8981, [retrieved on 20191112], DOI: 10.1016/J.CCA.2019.10.033
- VARIOUS: "Meeting Abstracts 23rd European workshop for rheumatology research", ARTHRITIS RESEARCH AND THERAPY, BIOMED CENTRAL, LONDON, GB, vol. 5, no. Suppl.1, 27 February 2003 (2003-02-27), pages S1 - S35, XP002293752, ISSN: 1478-6354, DOI: 10.1186/AR1010

## Description

La présente invention concerne une molécule hybride comprenant au moins un fragment Fc d'anticorps lié de façon covalente à au moins un peptide dérivé de fibrine comprenant un ou plusieurs résidu(s) citrullyl, les utilisations d'une telle molécule hybride, ainsi que son procédé de production.

### Contexte de l'invention

La polyarthrite rhumatoïde est le plus fréquent des rhumatismes inflammatoires ou arthrites, mais aussi la plus fréquente des maladies auto-immunes. La maladie est caractérisée par une inflammation chronique des articulations synoviales aboutissant à une destruction articulaire irréversible.

La présence d'auto-anticorps de classe G dirigés contre les protéines citrullinées et appelés auto-anticorps anti-protéines citrullinées (ACPA), est hautement spécifique de la polyarthrite rhumatoïde. Les sérums de patients qui contiennent des ACPA, les cellules lymphocytaires qui les expriment à leur membrane et les patients eux-mêmes, sont dits 'ACPA-positifs'. Plusieurs études ont démontré que ces ACPA sont au cœur des réactions auto-immunes propres à la maladie rhumatoïde et représentent ainsi une cible thérapeutique de choix.

Les cibles antigéniques des ACPA ont été caractérisées. Ils sont notamment dirigés spécifiquement contre des formes désiminées ou citrullinées des chaînes polypeptidiques α et β de la fibrine, protéine abondante dans le tissu synovial inflammatoire. Cette citrullination correspond à la désimination enzymatique des résidus arginyl des chaînes polypeptidiques, sous l'action de peptidyl-arginine désiminases (PAD).

Plus spécifiquement, les épitopes immunodominants reconnus par les ACPA sur les chaînes polypeptidiques α et β de la fibrine ont été caractérisés et publiés, notamment dans les demandes PCT/FR00/01857 ou PCT/FR2007/000758. Les cinq peptides citrullinés porteurs des épitopes immunodominants sont plus particulièrement les peptides nommés α36-50Cit (tel que représenté en SEQ ID NO : 5), α171-185Cit (tel que représenté en SEQ ID NO : 6), α501-515Cit (tel que représenté en SEQ ID NO : 14), α621-635Cit (tel que représenté en SEQ ID NO : 18) et β60-74Cit (tel que représenté en SEQ ID NO : 19). Les sérums de patients ACPA-positifs reconnaissent un ou plusieurs de ces cinq peptides.

Sécrétés dans le tissu synovial rhumatoïde par des plasmocytes locaux, les ACPA y présentent une concentration élevée, à proximité de leur principale cible, la fibrine citrullinée, qui y est, elle aussi, abondante sous forme de dépôts interstitiels. La fixation des ACPA sur ces dépôts et donc la formation de complexes immuns immobilisés, fixant à leur tour les facteurs rhumatoïdes, autres auto-anticorps associés à la polyarthrite rhumatoïde et eux aussi sécrétés par des plasmocytes locaux, déclenche une cascade d'évènements pro-inflammatoires.

La stimulation de cellules macrophagiques par ces macro-complexes immuns, essentiellement via leurs récepteurs Fcgamma membranaires, les conduit à sécréter des cytokines pro-inflammatoires et notamment du TNF-alpha qui a été identifié comme la principale cytokine responsable de l'inflammation rhumatoïde.

A ce jour il n'existe pas de traitement curatif de la polyarthrite rhumatoïde. Les traitements visent uniquement à soigner les poussées et/ou prévenir leur apparition.

Un objet de la présente invention est ainsi de fournir un traitement à la polyarthrite rhumatoïde.

Les ACPA sont oligoclonaux et donc sécrétés par seulement quelques clones plasmocytaires, résultant eux-mêmes de la différenciation de quelques clones lymphocytaires B.

En cas de polyarthrite rhumatoïde, des clones de lymphocytes B expriment à leur membrane des immunoglobulines porteuses de la spécificité ACPA (il s'agit de lymphocytes B ACPA-positifs), alors que les plasmocytes issus de la différenciation des clones lymphocytaires B ACPA-positifs (il s'agit de plasmocytes ACPA-positifs) sécrètent en abondance ces mêmes ACPA dans leur microenvironnement.

La présente invention repose sur les résultats des Inventeurs montrant qu'il est possible de cibler les clones lymphocytaires B exprimant des ACPA à leur surface et de les éliminer à l'aide d'une molécule hybride comprenant (i) au moins un peptide dérivé de fibrine ayant au moins un résidu citrullyl et (ii) un fragment Fc d'immunoglobuline humaine. Ces molécules hybrides cibleront spécifiquement les lymphocytes B ACPA-positifs (à l'aide du peptide dérivé de fibrine ayant au moins un résidu citrullyl, qui est reconnu par lesdits ACPA) qui seront ensuite éliminées, après fixation du fragment Fc sur les récepteurs Fc, par des macrophages (via phagocytose) et/ou des cellules NK (via cytotoxicité à médiation cellulaire dépendante des anticorps - ADCC), et/ou par activation de la cascade du complément.

En ciblant les clones lymphocytaires B exprimant des ACPA et les cellules qui se différencient en plasmocytes qui sécrètent eux-mêmes des ACPA, les molécules hybrides de l'invention visent ainsi à faire disparaître les ACPA de l'organisme des patients.

### Exposé de l'invention

### Molécule hybride selon l'invention

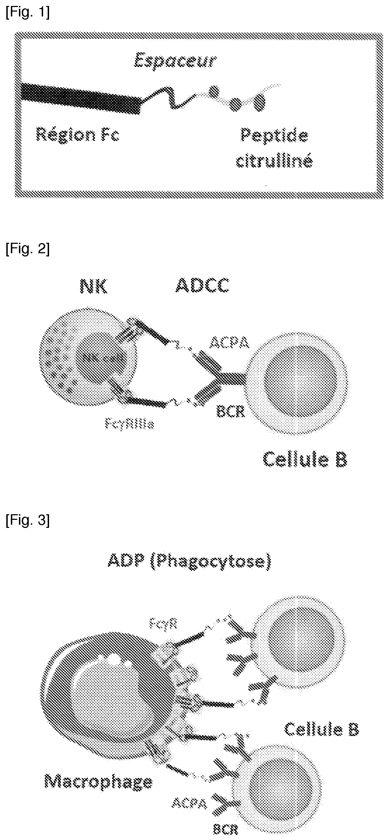

Dans un premier aspect, l'invention concerne une molécule hybride comprenant au moins un fragment Fc d'anticorps lié de façon covalente à au moins un peptide dérivé de fibrine ayant au moins un résidu citrullyl, au moins un espaceur étant optionnellement présent entre ledit fragment Fc et ledit peptide, ledit peptide étant reconnu par les auto-anticorps anti-protéines citrullinées et étant dérivé de tout ou partie de la séquence de la chaine α ou β d'une fibrine de vertébré, par substitution d'au moins un résidu arginyl par un résidu citrullyl. Le schéma d'une telle construction est présenté en Figure 1.

Selon l'invention, une « molécule hybride » s'entend d'une molécule ayant au moins deux composants de nature différente, en l'espèce le fragment Fc d'anticorps et ledit peptide.

Selon l'invention, un « fragment Fc » d'anticorps s'entend de la région constante d'une immunoglobuline à l'exclusion du premier domaine de région constante d'immunoglobuline (i.e. CH1-CL). Ainsi le fragment Fc fait référence à un homodimère, chaque monomère comprenant les deux derniers domaines constants des IgA, IgD, IgG (i.e. CH2 et CH3), ou les trois derniers domaines constants des IgE et IgM (i.e. CH2, CH3 et CH4).

Selon l'invention, l'expression « lié de façon covalente » s'entend d'une liaison covalente, c'est-à-dire une liaison chimique dans laquelle deux atomes se partagent deux électrons. Ladite liaison covalente peut être polaire ou non-polaire.

Selon l'invention, un « espaceur » est un agent de liaison qui permet de lier de façon covalente un fragment Fc d'anticorps audit peptide dérivé de fibrine ayant au moins un résidu citrullyl, tout en éloignant ledit fragment Fc dudit peptide (diminuant ainsi un éventuel encombrement stérique). Il peut s'agir de toute molécule, et notamment d'un peptide. De manière préférée, l'espaceur ne modifie pas les propriétés physico-chimiques de la molécule hybride.

La présence d'au moins un espaceur est avantageuse : elle permet de faciliter l'accessibilité indépendante des deux partenaires de la molécule hybride (le fragment Fc est plus facilement accessible pour se lier aux récepteurs Fc, de même que ledit peptide est plus facilement accessible pour se lier aux lymphocytes B ACPA-positifs), et/ou de stabiliser la molécule hybride, et/ou augmenter la solubilité de la molécule hybride.

Selon un mode de réalisation, la molécule hybride selon l'invention peut comprendre un ou plusieurs espaceurs. De préférence, la molécule hybride comprend un ou deux espaceurs. Selon un mode de réalisation, lorsqu'au moins un espaceur est présent dans ladite molécule hybride de l'invention, ledit fragment Fc est lié de façon covalente à un espaceur, ledit espaceur étant lui-même lié de façon covalente audit peptide. Selon un autre mode de réalisation, lorsqu'au moins deux espaceurs sont présents dans ladite molécule hybride de l'invention, ledit fragment Fc est lié de façon covalente à un premier espaceur, ledit premier espaceur étant lui-même lié de façon covalente à un deuxième espaceur et le deuxième espaceur est lui-même lié de façon covalente audit peptide. La liaison entre le fragment Fc et le peptide peut donc être directe, ou bien indirecte en présence d'espaceurs.

Selon un mode de réalisation, la molécule hybride selon l'invention peut comprendre au moins un peptide dérivé de fibrine ayant au moins un résidu citrullyl. Cela signifie que le fragment Fc peut être lié à un ou deux peptides. En effet, le fragment Fc comprend deux monomères, et le fragment Fc peut ainsi être lié de façon covalente à un peptide sur un seul des deux monomères, ou bien le fragment Fc peut être lié de façon covalente à un peptide sur chaque monomère. De préférence, lorsque deux peptides sont liés sur le fragment Fc, les deux peptides sont identiques, par exemple deux peptides de SEQ ID NO : 18 ou SEQ ID NO : 19.

Selon un mode de réalisation, ledit espaceur est un polymère contenant un ou plusieurs motifs de répétition contenant le groupe éther. Selon un mode de réalisation particulier, ledit espaceur est le polyéthylène glycol de formule PEGn, dans laquelle n représente un nombre entier compris entre 1 et 100, préférentiellement entre 1 et 10, et notamment 1, 2, 3, 4 ou 8. Selon l'invention ledit polyéthylène glycol peut être fonctionnalisé, par exemple avec un groupement amine (PEGn-amine tel que PEG-NH₂). Selon l'invention « un nombre entier compris entre 1 et 100 » représente toutes les valeurs entières comprises entre 1 et 100, i.e. ; 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 et 100.

Selon l'invention, l'expression « peptide dérivé de fibrine ayant au moins un résidu citrullyl » s'entend d'une molécule de fibrine ou de fibrinogène dans laquelle au moins un résidu arginyl a été substitué par un résidu citrullyl. Un « peptide dérivé de fibrine ayant au moins un résidu citrullyl » selon l'invention s'entend d'un peptide reconnu par les ACPA, et peut également être nommé un « peptide citrulliné ». De tels peptides peuvent être obtenus à partir de fragments de fibrine ou de fibrinogène naturels, recombinants ou de synthèse. De tels peptides peuvent également être directement synthétisés. Les acides aminés constituant le peptide peuvent être de série L ou D, de préférence de série L. Ladite substitution peut par exemple être réalisée par une étape de désimination enzymatique sous l'action de peptidyl-arginine désiminases (PAD). Un tel peptide peut aussi être obtenu en incorporant directement un ou plusieurs résidus citrullyl dans le peptide synthétisé. Un peptide selon l'invention se lie aux ACPA, et la liaison entre ledit peptide dérivé de fibrine ayant au moins un résidu citrullyl et un ACPA peut être vérifiée à l'aide d'un test ELISA ou tel que décrit dans la publication Sebbag M, Moinard N, Auger I, Clavel C, Arnaud J, Nogueira L, Roudier J, Serre G. Epitopes of human fibrin recognized by the rheumatoid arthritis-specific autoantibodies to citrullinated proteins. Eur J Immunol 36:2250-2263, 2006.

Selon l'invention, ledit peptide est dérivé de tout ou partie de la séquence de la chaine α ou β d'une fibrine de vertébré, par substitution d'au moins un résidu arginyl par un résidu citrullyl. Préférentiellement, ledit peptide est dérivé d'une séquence d'au moins 5 acides aminés consécutifs de la chaine α (notamment représentée par SEQ ID NO : 27) ou β (notamment représentée par SEQ ID NO : 28), d'une fibrine de vertébré. Encore plus particulièrement, ladite fibrine de vertébré est une fibrine de mammifère, de préférence humaine.

Selon un mode de réalisation, dans ladite molécule hybride selon l'invention, le peptide a une taille d'au moins 2 acides aminés consécutifs, 3 acides aminés consécutifs, 4 acides aminés consécutifs, de préférence 5 acides aminés consécutifs, et encore plus préférentiellement entre 5 et 25 acides aminés. Selon l'invention, « entre 5 et 25 » s'entend de toutes les valeurs : 5, 6, 7, 8, 9, 10, 11, 12, 13, 14, 15, 16, 17, 18, 19, 20, 21, 22, 23, 24 et 25. Préférentiellement, ledit peptide a une taille comprise entre 10 et 20 acides aminés, plus particulièrement 15 acides aminés.

Selon un mode de réalisation, dans ladite molécule hybride selon l'invention, le peptide est linéaire.

Selon un mode de réalisation, dans ladite molécule hybride selon l'invention, le peptide peut être modifié de façon à améliorer sa réactivité vis-à-vis des ACPA. A titre d'exemple, les peptides peuvent être cyclisés, les peptides peuvent être de type rétro (les acides aminés de série L sont enchainés selon une séquence inverse de celle du peptide à reproduire), ou de type rétro-inverso (les acides aminés sont de type D au lieu de la série naturelle L et sont enchainés selon une séquence inverse de celle du peptide à reproduire). Selon un mode de réalisation encore plus particulier, dans ladite molécule hybride selon l'invention, la fonction carboxyle (COOH) terminale dudit peptide est remplacée par une fonction carboxamide (CONH₂). De préférence, dans le peptide de SEQ ID NO : 12, la fonction carboxyle (COOH) terminale dudit peptide est remplacée par une fonction carboxamide (CONH₂). A titre d'exemple, le peptide β60-74Cit (tel que représenté en SEQ ID NO : 19) présente avantageusement une telle fonction carboxamide.

Selon un autre mode de réalisation, dans ladite molécule hybride selon l'invention, le peptide peut être modifié de façon à faciliter sa synthèse et/ou améliorer sa stabilité, par exemple par alkylation. Selon un mode de réalisation encore plus particulier, dans ladite molécule hybride selon l'invention, la fonction amine (NH₂) terminale dudit peptide est acétylée. De préférence, dans le peptide de SEQ ID NO : 1, la fonction amine terminale dudit peptide est acétylée. A titre d'exemple, le peptide α621-635Cit (tel que représenté en SEQ ID NO : 18) présente avantageusement une telle fonction acétyl sur son amine (NH₂) terminale.

Selon un mode de réalisation, dans ladite molécule hybride selon l'invention, les fonctions amine et carboxyle du peptide peuvent être sous forme du sel correspondant à l'acide ou à la base.

Selon un mode de réalisation, dans ladite molécule hybride selon l'invention, ledit peptide comprend au moins un résidu citrullyl, et est choisi dans le groupe constitué par :
a) un peptide défini par la séquence X₁PAPPPISGGGYX₂AX₃ (SEQ ID NO : 1) dans laquelle X₁, X₂, et X₃ représentent chacun un résidu citrullyl ou un résidu arginyl, et au moins l'un des résidus X1 ou X₂ ou X₃ est un résidu citrullyl ;
b) un peptide défini par la séquence GPX₁VVEX₂HQSACKDS (SEQ ID NO : 2) dans laquelle X₁ et X₂ représentent chacun un résidu citrullyl ou un résidu arginyl, et au moins l'un des résidus X₁ ou X₂ est un résidu citrullyl ;
c) un peptide défini par la séquence SGIGTLDGFX₁HX₂HPD (SEQ ID NO : 3) dans laquelle X₁ et X₂ représentent chacun un résidu citrullyl ou un résidu arginyl, et au moins l'un des résidus X₁ ou X₂ est un résidu citrullyl ;
d) un peptide défini par la séquence VDIDIKIX₁SCX₂GSCS (SEQ ID NO : 4) dans laquelle X₁ et X₂ représentent chacun un résidu citrullyl ou un résidu arginyl, et au moins l'un des résidus X₁ ou X₂ est un résidu citrullyl ;
e) un peptide défini par la séquence X₁GHAKSX₂PVX₃GIHTS (SEQ ID NO : 12) dans laquelle X₁, X₂ et X₃ représentent chacun un résidu citrullyl ou un résidu arginyl, et au moins l'un des résidus X₁ ou X₂ ou X₃ est un résidu citrullyl ;
f) un peptide comprenant au moins 5 acides aminés consécutifs, dont au moins un résidu citrullyl, de l'un des peptides a) à e) ci-dessus.

Selon un mode de réalisation particulier, ledit peptide comprend au moins un résidu citrullyl, et est choisi dans le groupe constitué par :
- un peptide défini par la séquence SEQ ID NO : 1 dans laquelle au moins un résidu choisi parmi X₁ ou X₂ ou X₃ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 2 dans laquelle au moins X₁ ou X₂ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 3 dans laquelle au moins X₁ ou X₂ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 4 dans laquelle au moins X₁ ou X₂ est un résidu citrullyl, ou un peptide comprenant un fragment d'au moins 5 acides aminés consécutifs de ladite séquence contenant ledit ou lesdits résidu(s) citrullyl ;
- un peptide défini par la séquence SEQ ID NO : 12 dans laquelle au moins un résidu choisi parmi X₁ ou X₂ ou X₃ est un résidu citrullyl, ou un peptide comprenant un fragment d'au moins 5 acides aminés consécutifs de ladite séquence contenant ledit ou lesdits résidu(s) citrullyl.

Selon un mode de réalisation encore plus particulier, dans ladite molécule hybride selon l'invention, ledit peptide comprend au moins un résidu citrullyl, et est choisi dans le groupe constitué par :
- un peptide défini par la séquence SEQ ID NO : 1 dans laquelle X₁, X₂, et X₃ sont des résidus citrullyl, ou un peptide d'au moins 15 acides aminés comprenant ladite séquence (il s'agit d'un peptide de SEQ ID NO : 19) ;
- un peptide défini par la séquence SEQ ID NO : 2 dans laquelle X₁ et X₂ sont des résidus citrullyl, ou un peptide comprenant un fragment d'au moins 5 acides aminés consécutifs de ladite séquence contenant lesdits résidus citrullyl (il s'agit d'un peptide de SEQ ID NO : 5) ;
- un peptide défini par la séquence SEQ ID NO : 3 dans laquelle X₁ et X₂ sont des résidus citrullyl, ou un peptide comprenant un fragment d'au moins 5 acides aminés consécutifs de ladite séquence contenant lesdits résidus citrullyl (il s'agit d'un peptide de SEQ ID NO : 14) ;
- un peptide défini par la séquence SEQ ID NO : 4 dans laquelle X₁ et X₂ sont des résidus citrullyl, ou un peptide comprenant un fragment d'au moins 5 acides aminés consécutifs de ladite séquence contenant lesdits résidus citrullyl (il s'agit d'un peptide de SEQ ID NO : 6);
- un peptide défini par la séquence SEQ ID NO : 12 dans laquelle X₁, X₂ et X₃ sont des résidus citrullyl, ou un peptide comprenant un fragment d'au moins 10 acides aminés consécutifs de ladite séquence contenant lesdits résidus citrullyl (il s'agit d'un peptide de SEQ ID NO : 18).

Selon un mode de réalisation encore plus particulier, dans ladite molécule hybride selon l'invention, ledit peptide est choisi dans le groupe constitué par : SEQ ID NO : 5 (α36-50cit_{38,42}), SEQ ID NO : 6 (α171-185cit_{178,181}), SEQ ID NO : 7 (α183-197cit_{186,190}), SEQ ID NO : 8 (α246-260cit₂₅₈), SEQ ID NO : 9 (α259-273cit_{263,271}), SEQ ID NO : 10 (α366-380cit₃₆₇), SEQ ID NO : 11 (α396-410cit₄₀₄), SEQ ID NO : 13 (α411-425cit₄₂₅), SEQ ID NO : 14 (α501-515cit_{510,512}), SEQ ID NO : 15 (α546-560cit₅₄₇), SEQ ID NO : 16 (α561-575cit₅₇₃), SEQ ID NO : 17 (α588-602cit₅₉₁), SEQ ID NO : 18 (α621-635cit_{621,627,630}), SEQ ID NO : 19 (β60-74cit_{60,72,74}), SEQ ID NO : 20 (β210-224cit₂₂₄), SEQ ID NO : 21 (β281-295cit_{285,294}), SEQ ID NO : 22 (β420-434cit₄₂₁) et SEQ ID NO : 23 (β433-447cit_{436,445}), plus particulièrement choisi dans le groupe constitué par : SEQ ID NO : 5 (α36-50cit_{38,42}), SEQ ID NO : 6 (α171-185cit_{178,181}), SEQ ID NO : 14 (α501-515cit_{510,512}), SEQ ID NO : 18 (α621-635cit_{621,627,630}) et SEQ ID NO : 19 (β60-74cit_{60,72,74}), encore plus particulièrement choisi parmi SEQ ID NO : 18 (α621-635cit_{621,627,630}) et SEQ ID NO : 19 (β60-74cit_{60,72,74}).

Selon un mode de réalisation, dans ladite molécule hybride selon l'invention, ledit fragment Fc est un fragment Fc humain, notamment d'IgG, plus particulièrement d'IgG1. L'IgG1 peut correspondre à n'importe quel variant allotypique par exemple G1m3 ou nG1m1. A titre d'exemple, le fragment Fc d'IgG1 est représenté par SEQ ID NO : 24, SEQ ID NO : 25 (Fc+Qtag) ou SEQ ID NO : 26 (Fc+Qtag bis).

Selon un mode de réalisation, dans ladite molécule hybride selon l'invention, ledit fragment Fc est de type sauvage ou muté. La ou les mutations peuvent viser à augmenter la demi-vie plasmatique, la diminuer, modifier les fonctions effectrices du fragment Fc. Selon un mode de réalisation encore plus particulier, ledit fragment Fc muté comprend au moins les mutations suivantes :
- L234A et L235A (LALA), ou
- L234A, L235A et P329G (LALAPG), ou
- G236A, S239D et I332E (GASDIE), ou
- G236A, S239D, A330L et I332E (GASDALIE), ou
- S239D, H268F, S324T et I332E (SDHFSTIE ou SDH),
la numérotation étant indiquée dans la séquence d'une IgG1 humaine selon l'index EU. De telles mutations sont notamment décrites dans l'article Bruhns and Jönsson, Immunol Rev. 2015 Nov;268(1):25-51. De préférence, lorsque la molécule hybride est utilisée en thérapie, ledit fragment Fc muté comprend au moins les mutations GASDIE, GASDALIE, ou SDH.

Selon un mode de réalisation, dans ladite molécule hybride selon l'invention, ledit fragment Fc présente un taux de fucosylation compris entre 0% à 100% des formes glycosylées. Selon l'invention « entre 0% et 100% » représente toutes les valeurs entières comprises entre 0 et 100, i.e ; 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 et 100%. Une faible fucosylation du fragment Fc provoque une forte réponse ADCC. C'est pourquoi selon un mode de réalisation particulier, ledit fragment Fc présente un taux de fucosylation compris entre 0% à 60% des formes glycosylées, notamment 50%, 40%, 30%, 20%, 10% ou 0%. Selon l'invention, le taux de fucosylation est défini comme la proportion moyenne de fucose portée par le fragment Fc, par rapport à la quantité maximale de fucose que peut porter un fragment Fc.

Le fragment Fc et le peptide ont chacun des extrémités N- et Cterminale. Le fragment Fc peut donc être lié via son extrémité N- ou Cterminale à l'extrémité N- ou Cterminale du peptide. Selon un mode de réalisation préféré, dans ladite molécule hybride selon l'invention, ladite liaison covalente se situe entre l'extrémité Cterminale dudit fragment Fc et l'extrémité Nterminale dudit peptide, ou entre l'extrémité Nterminale dudit fragment Fc et l'extrémité Nterminale dudit peptide. Selon un mode de réalisation, lorsqu'un espaceur est présent, l'espaceur peut être lié au fragment Fc via son extrémité N- ou Cterminale. Selon un autre mode de réalisation, lorsque deux espaceurs sont présents, le premier espaceur peut être lié au fragment Fc via son extrémité N- ou Cterminale et le deuxième espaceur peut être lié au peptide via son extrémité N- ou Cterminale, notamment Nterminale. Alternativement, ladite liaison covalente entre ledit fragment Fc et ledit peptide (éventuellement en présence d'un ou plusieurs espaceurs) peut être créée sur tout ou partie du fragment Fc. Selon l'invention « tout ou partie du fragment Fc » signifie que différents acides aminés constituant le fragment Fc peuvent être impliqués dans une liaison covalente avec ledit peptide.

Selon un mode de réalisation préféré, lorsqu'au moins un espaceur est présent dans ladite molécule hybride de l'invention, celui-ci permet de lier le fragment Fc à un azoture ou à un alcyne qui sera lui-même impliqué dans la liaison covalente avec ledit peptide. Selon un mode de réalisation, lorsqu'au moins un espaceur est présent dans ladite molécule hybride de l'invention, celui-ci peut également permettre de lier le peptide à un alcyne ou à un azoture qui sera lui-même impliqué dans la liaison covalente avec le fragment Fc. Selon un mode de réalisation préféré, la molécule hybride selon l'invention comprend au moins deux espaceurs : un premier espaceur qui permet de lier le fragment Fc à un azoture ou à un alcyne et un deuxième espaceur qui permet de lier le peptide à un azoture (lorsque le fragment Fc est lié à un alcyne) ou à un alcyne (lorsque le fragment Fc est lié à un azoture).

Selon un mode de réalisation selon l'invention, dans ladite molécule hybride selon l'invention, ledit fragment Fc :
- est couplé à au moins un azoture ou à un alcyne, tel qu'un cyclooctyne, et en particulier le DBCO, ou
- est lié à au moins un espaceur qui est lui-même couplé à un azoture ou à un alcyne, tel qu'un cyclooctyne, et en particulier le DBCO,

et ledit peptide est :
   - soit couplé à un azoture ou à un alcyne, tel qu'un cyclooctyne, et en particulier le DBCO,
   - soit lié à un espaceur qui est lui-même couplé à un azoture ou à un alcyne, tel qu'un cyclooctyne, et en particulier le DBCO,
la liaison covalente entre ledit fragment Fc et ledit peptide, éventuellement en présence d'un ou plusieurs espaceurs, étant créée entre l'azoture et l'alcyne.

Selon un mode de réalisation selon l'invention, dans ladite molécule hybride selon l'invention,
- le fragment Fc est couplé à au moins un azoture et ledit peptide est couplé à un alcyne, tel qu'un cyclooctyne, et en particulier le DBCO, ou
- le fragment Fc est couplé à au moins un alcyne, tel qu'un cyclooctyne, et en particulier le DBCO, et ledit peptide est couplé à un azoture,
la liaison covalente entre ledit fragment Fc et ledit peptide étant créée entre l'azoture et l'alcyne.

Selon un mode de réalisation selon l'invention, dans ladite molécule hybride selon l'invention :
- le fragment Fc est couplé à au moins un azoture et ledit peptide est lié à un espaceur, lui-même couplé à un alcyne, tel qu'un cyclooctyne, et en particulier le DBCO ou
- le fragment Fc est couplé à au moins un alcyne, tel qu'un cyclooctyne, et en particulier le DBCO, et ledit peptide est lié à un espaceur, lui-même couplé à un azoture ou
- le fragment Fc est lié à au moins un espaceur, lui-même couplé à un azoture, et ledit peptide est couplé à un alcyne, tel qu'un cyclooctyne, et en particulier le DBCO ou
- le fragment Fc est lié à au moins un espaceur, lui-même couplé à un alcyne, tel qu'un cyclooctyne, et en particulier le DBCO, et ledit peptide est couplé à un azoture ou
- le fragment Fc est lié à au moins un espaceur, lui-même couplé à un azoture, et ledit peptide est lié à un espaceur, lui-même couplé à un alcyne, tel qu'un cyclooctyne, et en particulier le DBCO ou
- le fragment Fc est lié à au moins un espaceur, lui-même couplé à un alcyne, tel qu'un cyclooctyne, et en particulier le DBCO, et ledit peptide est lié à un espaceur, lui-même couplé à un azoture,
la liaison covalente entre ledit fragment Fc et ledit peptide, en présence d'un ou plusieurs espaceurs, étant créée entre l'azoture et l'alcyne.

Selon un mode de réalisation, dans les molécules décrites ci-dessus, plus particulièrement de la page 8, ligne 21 à la page 9, ligne 17, l'espaceur utilisé est de préférence un espaceur PEGn, n représentant plus particulièrement un entier compris entre 1 et 10. Lorsque la molécule hybride selon l'invention comprend au moins deux espaceurs PEGn, les n des deux espaceurs peuvent être identiques ou différents. Par exemple le premier espaceur peut être un PEG₂ et le deuxième espaceur peut être un PEG₃ ou PEG₄.

Selon un mode de réalisation particulièrement préféré, dans les molécules décrites ci-dessus, plus particulièrement de la page 8, ligne 21 à la page 9, ligne 17, le peptide est dérivé de fibrine ou fibrinogène humain et le fragment Fc est un Fc humain, de préférence d'IgG1 humaine.

Selon l'invention, la création de la liaison covalente entre l'azoture et l'alcyne correspond à une étape dite de « chimie-click », la partie N₃ de l'azoture réagissant avec un alcyne. L'azoture s'entend des sels de l'acide azothydrique HN₃, ou des azotures organiques dans lesquels un des atomes d'azote est lié de façon covalente avec un atome de carbone d'un composé organique (par exemple l'azoture de méthyle CH₃N₃). Préférentiellement l'azoture est représenté par la formule N₃. L'alcyne s'entend des molécules ayant pour formule générale CₙH₂ₙ₋₂, et qui sont caractérisées par la présence d'au moins une triple liaison. Préférentiellement l'alcyne est un cyclooctyne, encore plus préférentiellement le dibenzocyclooctyne (DBCO).

Le fragment Fc, ledit peptide et éventuellement le(s)dit(s) espaceur(s), sont couplés à l'alcyne ou à l'azoture par toute technique de couplage moléculaire classiquement utilisée (telle qu'une conjugaison). Toute technique peut également être utilisée pour lier de façon covalente le fragment Fc à l'espaceur et/ou le peptide à l'espaceur.

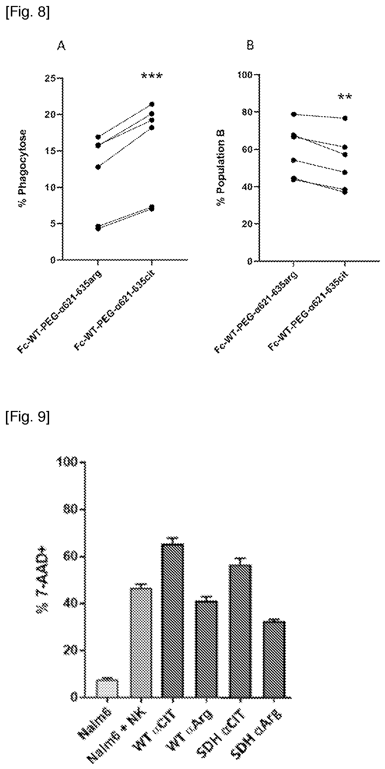

Plus particulièrement, une technique de conjugaison s'entend d'une conjugaison enzymatique ou d'une conjugaison chimique. Une conjugaison enzymatique s'entend par exemple d'une conjugaison à l'aide d'une transglutaminase qui catalyse la formation de liaisons covalentes entre des groupes amines libres et des résidus de glutamine ou de lysine ou encore à l'aide d'une transpeptidase telle que la sortase. Pour de plus amples informations concernant la conjugaison enzymatique, voir par exemple la demande de brevet US20160361434 ou US20170313787, ou encore la publication Ohtsuka et al., Bioscience, Biotechnology, and Biochemistry Volume 64, 2000 - Issue 12, Comparison of Substrate Specificities of Transglutaminases Using Synthetic Peptides as Acyl donors. Le substrat de la transglutaminase est par exemple un peptide comportant un résidu glutamyl (un Qtag), tel que représenté par SEQ ID NO : 29 (LLQG). Une conjugaison chimique s'entend par exemple d'une liaison covalente entre une cystéine isolée ou participant à un pont disulfure après réduction de celui-ci et par exemple un maléimide. Un exemple d'une telle conjugaison est représenté en Figure 9. Dans cet exemple, le fragment Fc comprend un peptide Qtag et ledit fragment Fc est lié à un espaceur (lui-même couplé à un azoture), grâce à l'action de la transglutaminase qui va créer une liaison covalente entre le résidu glutamyl du Qtag et le groupe NH₂ porté par l'espaceur PEGn.

Selon l'invention, le terme « couplé » ou « couplage moléculaire » s'entend de l'établissement d'une liaison covalente, ainsi le fragment Fc et/ou le peptide et/ou l'espaceur est lié de façon covalente à un alcyne ou un azoture. Le terme « lié » s'entend également d'une liaison covalente. Ainsi, à titre d'exemple, l'expression « le fragment Fc est couplé à un azoture et ledit peptide est lié à un espaceur, lui-même couplé à un alcycne, tel qu'un cyclooctyne, et en particulier le DBCO » peut également se lire « le fragment Fc est lié de façon covalente à un azoture et ledit peptide est lié de façon covalente à un espaceur, ledit espaceur étant lui-même lié de façon covalente à un alcycne, tel qu'un cyclooctyne, et en particulier le DBCO ».

Selon un mode de réalisation selon l'invention, ladite molécule hybride est représentée par :
- un fragment Fc de type sauvage qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
- un fragment Fc comprenant les mutations S239D, H268F, S324T et I332E qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
- un fragment Fc comprenant les mutations L234A, L235A et P329G qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
- un fragment Fc de type sauvage qui est lié à au moins un espaceur PEGn qui est lui-même couplé à un cyclooctyne lié de façon covalente à un azoture couplé à un espaceur PEGn lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations S239D, H268F, S324T et I332E qui est lié à au moins un espaceur PEGn qui est lui-même couplé à un cyclooctyne lié de façon covalente à un azoture couplé à un espaceur PEGn lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations L234A, L235A et P329G qui est lié à au moins un espaceur PEGn qui est lui-même couplé à un cyclooctyne lié de façon covalente à un azoture couplé à un espaceur PEGn lui-même lié à un peptide représenté par SEQ ID NO : 19 (β60-74cit_{60,72,74}) ou SEQ ID NO : 18 (α621-635cit_{621,627,630}),
- un fragment Fc de type sauvage qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations S239D, H268F, S324T et I332E qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations L234A, L235A et P329G qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations G236A, S239D et I332E, qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations G236A, S239D, A330L et I332E qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations G236A, S239D et 1332E qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
- un fragment Fc comprenant les mutations G236A, S239D, A330L et I332E qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
n représentant de préférence un entier entre 1 et 10, plus particulièrement 1, 2, 3, 4 ou 8.

Selon un mode de réalisation selon l'invention, ladite molécule hybride est représentée par :
- un fragment Fc de type sauvage qui est lié à au moins un espaceur PEGn qui est lui-même couplé à un azoture lié de façon covalente à un cyclooctyne qui est couplé à un peptide représenté par SEQ ID NO : 19 (B60-74cit_{60,72,74}) ou SEQ ID NO : 18 (α621-635cit_{621,627,630}),
- un fragment Fc comprenant les mutations S239D, H268F, S324T et I332E qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
- un fragment Fc comprenant les mutations L234A, L235A et P329G qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}) ou SEQ ID NO : 18 (α621-635cit_{621,627,630}),
- un fragment Fc de type sauvage qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
- un fragment Fc comprenant les mutations S239D, H268F, S324T et I332E qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
n représentant de préférence un entier entre 1 et 10, plus particulièrement 1, 2, 3, 4 ou 8.

Selon un mode de réalisation encore plus particulier selon l'invention, ladite molécule hybride est représentée par :
- un fragment Fc de type sauvage qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}),
- un fragment Fc comprenant les mutations S239D, H268F, S324T et I332E qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}),
- un fragment Fc comprenant les mutations L234A, L235A et P329G qui est lié à au moins un espaceur PEGn qui est lui-même couplé à un azoture lié de façon covalente à un cyclooctyne qui est couplé à un peptide représenté par SEQ ID NO : 19 (β60-74cit_{60,72,74}),
- un fragment Fc de type sauvage qui est lié à un espaceur PEGn qui est lui-même couplé à au moins un azoture lié de façon covalente à un cyclooctyne couplé à un espaceur PEGn qui est lui-même lié à un peptide représenté par SEQ ID NO : 19 (B60-74cit_{60,72,74}) ou SEQ ID NO : 18 (α621-635cit_{621,627,630}),
- un fragment Fc comprenant les mutations S239D, H268F, S324T et I332E qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 (β60-74cit_{60,72,74}) ou SEQ ID NO : 18 (α621-635cit_{621,627,630}),
n représentant de préférence un entier entre 1 et 10, plus particulièrement 1, 2, 3, 4 ou 8.

### Utilisation des molécules hybrides selon l'invention

Dans un second aspect, la présente invention concerne également une molécule hybride telle que définie précédemment, pour son utilisation comme médicament.

Plus particulièrement, selon l'invention, lesdites molécules hybrides sont destinées à cibler et à lyser dans l'organisme des patients, par ADCC et/ou phagocytose et/ou activation du complément, toutes les cellules B « ACPA-positives » (i.e. les lymphocytes B qui expriment à leur surface les auto-anticorps anti-protéines citrullinées (ACPA)). En effet, la molécule hybride selon l'invention se lie aux lymphocytes B « ACPA-positifs » grâce au peptide dérivé de fibrine ayant au moins un résidu citrullyl : il s'agit de la cible épitopique dudit ACPA. La molécule hybride selon l'invention se lie aussi aux cellules permettant de détruire les lymphocytes B « ACPA-positifs » grâce à son fragment Fc, ligand naturel des récepteurs Fc (par exemple Fc-gamma récepteur (FcγR) si le fragment Fc est issu d'une IgG), présents notamment à la surface des macrophages mais aussi des cellules NK ("Natural Killers").

Selon un mode de réalisation particulier, l'invention concerne une molécule hybride, telle que définie précédemment pour son utilisation dans le traitement des maladies auto-immunes associées à la production d'auto-anticorps anti-protéines citrullinées, en particulier le syndrome de Gougerot-Sjögren et la polyarthrite rhumatoïde. Dans ce mode de réalisation, les formes sévères desdites maladies auto-immunes associées à la production d'auto-anticorps anti-protéines citrullinées sont ciblées, de même que diverses formes dites « frontières » avec d'autres arthrites chroniques, telles que l'arthrite psoriasique ou le lupus érythémateux disséminé.

Selon un mode de réalisation, l'invention concerne également une composition pharmaceutique comprenant une molécule hybride selon l'une quelconque des revendications précédentes, en combinaison avec un véhicule pharmaceutiquement acceptable.

Selon l'invention « un véhicule pharmaceutiquement acceptable » s'entend de toute formulation rendant la composition apte à être administrée à un patient, sous n'importe quelle forme galénique.

Comme indiqué ci-dessus, la présente invention vise à détruire les lymphocytes B « ACPA-positifs ». Cependant, les lymphocytes B « ACPA-positifs » qui se sont différenciés en plasmocytes sécréteurs d'ACPA ne sont plus ciblés par les molécules hybrides précédemment décrites. Selon un mode de réalisation particulier, la présente invention concerne ainsi une molécule hybride telle que précédemment décrite, pour son utilisation comme médicament, en combinaison avec une deuxième molécule hybride, ladite deuxième molécule hybride comprenant au moins un peptide dérivé de fibrine ayant au moins un résidu citrullyl, ledit peptide étant lié de façon covalente à au moins un anticorps ou à au moins un fragment d'anticorps, ledit anticorps ou fragment étant capable de se lier à CD38 et/ou CD138, un ou plusieurs espaceurs étant optionnellement présents entre ledit peptide et ledit anticorps ou ledit fragment. Une telle thérapie de combinaison permet de cibler et détruire spécifiquement les clones plasmocytaires et lymphocytaires B ACPA-positifs, et ainsi de faire disparaître les ACPA de l'organisme des patients. L'anticorps utilisé est de préférence un anticorps monoclonal.

Selon un mode de réalisation particulier, dans cette deuxième molécule hybride, le fragment s'entend d'une portion d'anticorps comprenant un site de liaison à l'antigène ou une région variable de l'anticorps. De tels fragments d'anticorps sont par exemple Fab, F(ab')2, Fv, dsFv, scFv, les fragments simples chaines tels que les fragments VH ou VL isolés, les VHH de camélidés, les VNAR de poisson cartilagineux, ou encore des anticorps multispécifiques composés de différents fragments, tels que des anticorps bi-spécifiques, ou encore des « nanobodies » « diabodies », « triabodies », « tetrabodies », ou des scFv en tandem, ... Ces fragments sont bien connus de l'homme du métier. De plus amples informations concernant ces fragments et constructions sont par exemple décrites dans la demande internationale WO2017137579, Bird et al., 1988 Science 242:423-426; and Huston et al., 1988 Proc. Natl. Acad. Sci. 85:5879-5883, ou encore Nelson, mAbs 2:1, 77-83; January/February 2010. De préférence le fragment est un Fab ou F(ab')2.

Selon un mode de réalisation particulier, dans cette deuxième molécule hybride ledit anticorps ou fragment F(ab')2 est un anticorps ou fragment F(ab')2 bispécifique dirigé contre CD38 et une autre cible plasmocytaire. Alternativement, dans cette deuxième molécule hybride ledit anticorps ou fragment F(ab')2 est un anticorps ou fragment F(ab')2 bispécifique dirigé contre CD138 et une autre cible plasmocytaire. Selon l'invention, une « autre cible plasmocytaire » s'entend de toute molécule étant exprimée à la surface des plasmocytes.

Selon un autre mode de réalisation particulier, dans cette deuxième molécule hybride ledit anticorps ou fragment F(ab')₂ est un anticorps ou fragment F(ab')₂ bispécifique dirigé contre CD38 et CD138.

Selon ce mode de réalisation particulier de l'invention, la première et la deuxième molécule hybride sont administrées de façon simultanée, séparée ou étalée dans le temps.

Selon un autre mode de réalisation, la molécule hybride selon l'invention peut être couplée à au moins un radioisotope ou à au moins un fluorochrome, tels que A488 ou A647. De telles molécules peuvent avantageusement être utilisées comme outils moléculaires traceurs.

Selon un autre mode de réalisation, l'invention concerne ainsi l'utilisation *in vitro* ou *ex vivo* d'une molécule hybride comprenant au moins un fragment Fc d'anticorps lié de façon covalente à au moins un peptide dérivé de fibrine ayant au moins un résidu citrullyl, au moins un espaceur étant optionnellement présent entre ledit fragment Fc et ledit peptide, comme outil moléculaire. De telles constructions peuvent notamment être utilisées pour analyser la fixation des molécules hybrides aux ACPA et aux récepteurs Fc des macrophages et des cellules NK, ainsi que pour analyser la réactivité des macrophages et des cellules NK à la fixation des hybrides suivie du pontage de ceux-ci par les ACPA.... Les radioisotopes et/ou fluorochromes sont de préférence couplés sur le fragment Fc, encore plus particulièrement au niveau du Qtag (si présent) ou au niveau des lysines.

### Procédé de production des molécules hybrides selon l'invention

Dans un autre aspect, l'invention concerne également un procédé permettant d'obtenir une molécule hybride telle que définie précédemment.

Selon un mode de réalisation, l'invention concerne ainsi un procédé de production d'une molécule hybride telle que définie précédemment, comprenant les étapes suivantes :
- (i) obtention d'un azoture couplé à un fragment Fc ou obtention d'un alcyne couplé à un fragment Fc,
- (ii) obtention d'un alcyne couplé à un peptide ou obtention d'un azoture couplé à un peptide,
- (iii) réalisation de la liaison covalente entre l'azoture et l'alcyne,
- l'étape (i) pouvant être réalisée avant ou après l'étape (ii), ou bien de façon concomitante.

Selon un mode de réalisation, l'invention concerne ainsi un procédé de production d'une molécule hybride telle que définie précédemment, comprenant les étapes suivantes :
- (i) couplage d'au moins un azoture et d'un fragment Fc, éventuellement en présence d'au moins un espaceur, ou couplage d'au moins un alcyne et d'au moins un fragment Fc, éventuellement en présence d'un espaceur,
- (ii) couplage d'un alcyne et d'un peptide, éventuellement en présence d'un espaceur, ou couplage d'un azoture et d'un peptide, éventuellement en présence d'un espaceur,
- (iii) réalisation de la liaison covalente entre l'azoture et l'alcyne,
- l'étape (i) pouvant être réalisée avant ou après l'étape (ii), ou bien de façon concomitante.

Selon un autre mode de réalisation, l'invention concerne ainsi un procédé de production d'une molécule hybride telle que définie précédemment, comprenant les étapes suivantes :
- (i) couplage d'au moins un azoture sur chaque monomère du fragment Fc, éventuellement en présence d'au moins un espaceur, ou couplage d'au moins un alcyne sur chaque monomère du fragment Fc, éventuellement en présence d'un espaceur,
- (ii) couplage d'un alcyne et d'un peptide, éventuellement en présence d'un espaceur, ou couplage d'un azoture et d'un peptide, éventuellement en présence d'un espaceur,
- (iii) réalisation de la ou les liaison(s) covalente(s) entre l'azoture et l'alcyne,
- l'étape (i) pouvant être réalisée avant ou après l'étape (ii), ou bien de façon concomitante.

Les séquences de l'invention sont représentées dans le Tableau 1 ci-après.

**[Tableau 1]. Tableau récapitulatif des séquences de l'invention**

| Numéro de la séquence / Nom de la séquence le cas échéant | Séquence |
|---|---|
| SEQ ID NO : 1 | X₁PAPPPISGGGYX₂AX₃ |
| SEQ ID NO : 2 | GPX₁VVEX₂HQSACKDS |
| SEQ ID NO : 3 | SGIGTLDGFX₁HX₂HPD |
| SEQ ID NO : 4 | VDIDIKIX₁SCX₂GSCS |
| SEQ ID NO : 12 | X₁GHAKSX₂PVX₃GIHTS |
| SEQ ID NO : 5 / α36-50cit_{38,42} | GPXVVEXHQSACKDS, X représentant un résidu citrullyl |
| SEQ ID NO : 6 / α171-185cit_{178,181} | VDIDIKIXSCXGSCS, X représentant un résidu citrullyl |
| SEQ ID NO : 7 / α183-197cit_{186,190} | SCSXALAXEVDLKDY, X représentant un résidu citrullyl |
| SEQ ID NO : 8/ α246-260cit₂₅₈ | PEWKALTDMPQMXME, X représentant un résidu citrullyl |
| SEQ ID NO : 9 / α259-273cit_{263,271} | MELEXPGGNEITXGG, X représentant un résidu citrullyl |
| SEQ ID NO : 10 / α366-380cit₃₆₇ | EXGSAGHWTSESSVS, X représentant un résidu citrullyl |
| SEQ ID NO : 11 / α396-410cit₄₀₄ | DSPGSGNAXPNNPDW, X représentant un résidu citrullyl |
| SEQ ID NO : 13 / α411-425cit₄₂₅ | GTFEEVSGNVSPGTX, X représentant un résidu citrullyl |
| SEQ ID NO : 14 / α501-515cit_{510,512} | SGIGTLDGFXHXHPD, X représentant un résidu citrullyl |
| SEQ ID NO : 15 / a546-560cit₅₄₇ | SXGSESGIFTNTKES, X représentant un résidu citrullyl |
| SEQ ID NO : 16 / α561-575cit₅₇₃ | SSHHPGIAEFPSXGK, X représentant un résidu citrullyl |
| SEQ ID NO : 17 / α588-602cit₅₉₁ | SYNXGDSTFESKSYK, X représentant un résidu citrullyl |
| SEQ ID NO : 18 / α621-635cit_{621,627,630} | XGHAKSXPVXGIHTS, X représentant un résidu citrullyl |
| SEQ ID NO : 19 / B60-74cit_{60,72,74} | XPAPPPISGGGYXAX, X représentant un résidu citrullyl |
| SEQ ID NO : 20 / β210-224cit₂₂₄ | QKLESDVSAQMEYCX, X représentant un résidu citrullyl |
| SEQ ID NO : 21 / β281-295cit_{285,294} | VIQNXQDGSVDFGXK, X représentant un résidu citrullyl |
| SEQ ID NO : 22 / β420-434cit₄₂₁ | PXKQCSKEDGGGWWY, X représentant un résidu citrullyl |
| SEQ ID NO : 23 / β433-447cit_{436,445} | WYNXCHAANPNGXYY, X représentant un résidu citrullyl |
| SEQ ID NO : 24 / Fragment Fc | APELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLY SKLTVDKSRWQQGNVFSCSVMHEALHNHYTQKSLSLSPGK |
| SEQ ID NO : 25 / Fragment Fc + Qtag | LLQGARSDATHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPG K |
| SEQ ID NO : 26 / Fragment Fc + Qtag bis | AHGHGHGLLQGARSDATHTCPPCPAPELLGGPSVFLFPPKPKDTLMISRTPEVTCVVVD VSHEDPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAVEWE SNGQPENNYKTTPPVLDSDGSFFLYSKLTVDKSRWQQGNVFSCSVMHEALHNHYTQK SLSLSPGK |
| SEQ ID NO : 27 / Chaine α de fibrine | GPRVVERHQSACKDSDWPFCSDEDWNYKCPSGCRMKGLIDEVNQDFTNRINKLKNSL FEYQKNNKDSHSLTTNIMEILRGDFSSANNRDNTYNRVSEDLRSRIEVLKRKVIEKVQHI QLLQKNVRAQLVDMKRLEVDIDIKIRSCRGSCSRALAREVDLKDYEDQQKQLEQVIAKDL LPSRDRQHLPLIKMKPVPDLVPGNFKSQLQKVPPEWKALTDMPQMRMELERPGGNEIT RGGSTSYGTGSETESPRNPSSAGSWNSGSSGPGSTGNRNPGSSGTGGTATWKPGSS GPGSTGSWNSGSSGTGSTGNQNPGSPRPGSTGTWNPGSSERGSAGHWTSESSVSG STGQWHSESGSFRPDSPGSGNARPNNPDWGTFEEVSGNVSPGTRREYHTEKLVTSK GDKELRTGKEKVTSGSTTTTRRSCSKTVTKTVIGPDGHKEVTKEVVTSEDGSDCPEAM DLGTLSGIGTLDGFRHRHPDEAAFFDTASTGKTFPGFFSPMLGEFVSETESRGSESGIF TNTKESSSHHPGIAEFPSRGKSSSYSKQFTSSTSYNRGDSTFESKSYKMADEAGSEAD HEGTHSTKRGHAKSRPVRGIHTS |
| SEQ ID NO : 28 / | GHRPLDKKREEAPSLRPAPPPISGGGYRARPAKAAATQKKVERKAPDAGGCLHADPDL GVLCPTGCQLQEALLQQERPIRNSVDELNNNVEAVSQTSSSSFQYMYLLKDLWQKRQK |
| Chaine β de fibrine | QVKDNENVVNEYSSELEKHQLYIDETVNSNIPTNLRVLRSILENLRSKIQKLESDVSAQM EYCRTPCTVSCNIPVVSGKECEEIIRKGGETSEMYLIQPDSSVKPYRVYCDMNTENGGW TVIQNRQDGSVDFGRKWDPYKQGFGNVATNTDGKNYCGLPGEYWLGNDKISQLTRMG PTELLIEMEDWKGDKVKAHYGGFTVQNEANKYQISVNKYRGTAGNALMDGASQLMGE NRTMTIHNGMFFSTYDRDNDGWLTSDPRKQCSKEDGGGWWYNRCHAANPNGRYYW GGQYTWDMAKHGTDDGVVWMNWKGSWYSMRKMSMKIRPFFPQQ |
| SEQ ID NO : 29 (Exemple de Qtag) | LLQG |
| SEQ ID NO : 30 | MFSMRIVCLVLSVVGTAWTADSGEGDFLAEGGGVRGPRVVERHQSACKDSDWPFCSD EDWNYKCPSGCRMKGLIDEVNQDFTNRINKLKNSLFEYQKNNKDSHSLTTNIMEILRGD FSSANNRDNTYNRVSEDLRSRIEVLKRKVIEKVQHIQLLQKNVRAQLVDMKRLEVDIDIKI RSCRGSCSRALAREVDLKDYEDQQKQLEQVIAKDLLPSRDRQHLPLIKMKPVPDLVPGN FKSQLQKVPPEWKALTDMPQMRMELERPGGNEITRGGSTSYGTGSETESPRNPSSAG SWNSGSSGPGSTGNRNPGSSGTGGTATWKPGSSGPGSTGSWNSGSSGTGSTGNQN PGSPRPGSTGTWNPGSSERGSAGHWTSESSVSGSTGQWHSESGSFRPDSPGSGNA RPNNPDWGTFEEVSGNVSPGTRREYHTEKLVTSKGDKELRTGKEKVTSGSTTTTRRSC SKTVTKTVIGPDGHKEVTKEVVTSEDGSDCPEAMDLGTLSGIGTLDGFRHRHPDEAAFF DTASTGKTFPGFFSPMLGEFVSETESRGSESGIFTNTKESSSHHPGIAEFPSRGKSSSY SKQFTSSTSYNRGDSTFESKSYKMADEAGSEADHEGTHSTKRGHAKSRPVRGIHTS MKRMVSWSFHKLKTMKHLLLLLLCVFLVKSQGVNDNEEGFFSARGHRPLDKKREEAPS LRPAPPPISGGGYRARPAKAAATQKKVERKAPDAGGCLHADPDLGVLCPTGCQLQEAL LQQERPIRNSVDELNNNVEAVSQTSSSSFQYMYLLKDLWQKRQKQVKDNENVVNEYS SELEKHQLYIDETVNSNIPTNLRVLRSILENLRSKIQKLESDVSAQMEYCRTPCTVSCNIP VVSGKECEEIIRKGGETSEMYLIQPDSSVKPYRVYCDMNTENGGWTVIQNRQDGSVDF GRKWDPYKQGFGNVATNTDGKNYCGLPGEYWLGNDKISQLTRMGPTELLIEMEDWKG DKVKAHYGGFTVQNEANKYQISVNKYRGTAGNALMDGASQLMGENRTMTIHNGMFFS TYDRDNDGWLTSDPRKQCSKEDGGGWWYNRCHAANPNGRYYWGGQYTWDMAKHG TDDGVVWMNWKGSWYSMRKMSMKIRPFFPQQ |
| Chaine α de fibrinogène | |
| SEQ ID NO : 31 | |
| Chaine β de fibrinogène | |

D'autres caractéristiques, détails et avantages de l'invention apparaîtront à la lecture des Figures annexées et des exemples qui illustrent l'invention et ne visent en aucun cas à la limiter.

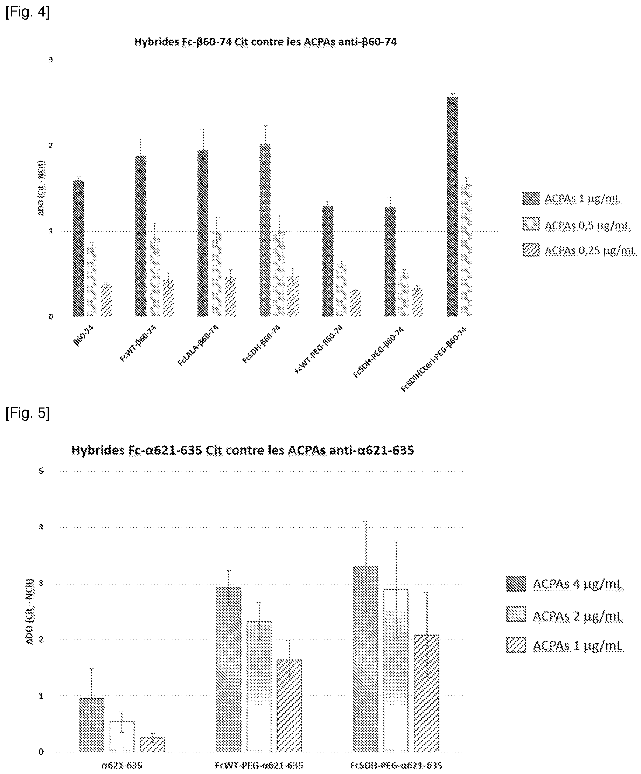

Dans les molécules hybrides exemplifiées, c'est l'extrémité Nterminale du fragment Fc qui est liée à un espaceur PEGn (sauf dans le cas indiqué Cter-PEG (voir la Figure 4)), et c'est toujours l'extrémité Nterminale dudit peptide qui est lié au cycloctyne DBCO ou à un deuxième espaceur PEGn le cas échéant. De plus, dans les molécules hybrides exemplifiées, dans l'espaceur PEGn lié au fragment Fc, n représente plus particulièrement 3 ou 4, et dans l'espaceur PEGn lié au peptide, l'espaceur PEGn représente 3 ou 8, bien que toute autre valeur de n, notamment entre 1 et 10, puisse être utilisée.

Dans les exemples ci-après une cellule « armée ou pré-armée » s'entend d'une cellule sur laquelle est fixée une molécule hybride selon l'invention. Ainsi, la molécule hybride est liée au récepteur Fc de la cellule grâce à son fragment Fc.

### Brève description des Figures

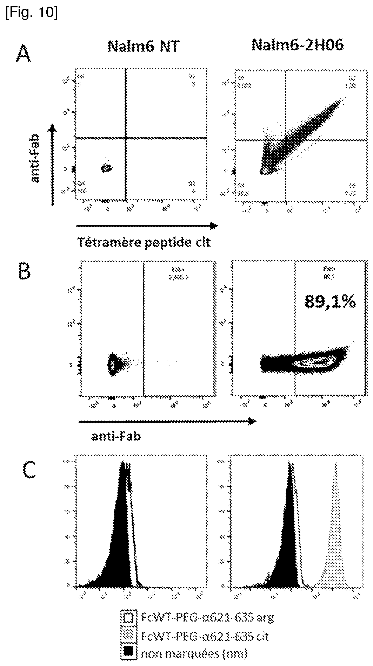

**Fig. 1**
   [Fig. 1] **représente le schéma d'un exemple de molécule hybride selon l'invention.**
   Un espaceur (qui est optionnel) est représenté entre le fragment Fc d'anticorps et le peptide dérivé de fibrine ayant un résidu citrullyl (dénommé « peptide citrulliné »).
**Fig. 2**
   [Fig. 2] **représente un lymphocyte B exprimant à sa surface des ACPA transmembranaires liés à une molécule hybride selon l'invention, ladite molécule hybride étant elle-même liée par le fragment Fc à une cellule NK.**
   La cellule NK va ainsi pouvoir détruire le lymphocyte B par ADCC. Plus spécifiquement, cette figure montre une cellule B (ou lymphocyte B) qui exprime à sa surface un BCR de type ACPA et qui interagit spécifiquement avec le peptide citrulliné porté par la molécule hybride. L'engagement du fragment Fc porté par la même molécule hybride au FcyRllla (CD16a) exprimé à la surface d'une cellule NK va activer l'ADCC et induire la destruction spécifique du lymphocyte B ACPA-positif. (ACPA, Anti-Citrullinated Protein Autoantibodies; ADCC : Antibody-Dependent Cell Cytotoxicity; BCR, B-Cell Receptor ; Fc γ R, Fc-gamma Receptor; NK cell, Natural Killer cell).
**Fig. 3**
   [Fig. 3] **représente un lymphocyte B exprimant à sa surface des ACPA transmembranaires liés à une molécule hybride selon l'invention, ladite molécule hybride étant elle-même liée par le fragment Fc à un macrophage.**
   Le macrophage va ainsi pouvoir détruire le lymphocyte B par phagocytose. Plus spécifiquement, cette Figure montre des cellules B (ou lymphocytes B) qui expriment à leur surface un BCR de type ACPA et qui interagissent spécifiquement avec des peptides citrullinés portés par les molécules hybrides. L'engagement des fragments Fc portés par ces mêmes molécules hybrides à différents FcyRs exprimés à la surface d'un macrophage va activer l'ADP, c'est-à-dire la phagocytose, et induire la destruction spécifique des lymphocytes B ACPA-positifs. (ACPA, Anti-Citrullinated Protein Autoantibodies; ADP, Antibody-Dependent Phagocytosis; BCR, B-Cell Receptor; Fc γ R, Fc-gamma Receptor).
**Fig. 4**
   [Fig. 4] **représente la réactivité de plusieurs molécules hybrides comprenant le peptide de SEQ ID NO : 19 (β60-74cit_{60,72,74}) vis-à-vis des ACPA.**
   β60-74 représente le peptide de SEQ ID NO : 19. Fc-WT-β60-74 représente un fragment Fc de type sauvage (Wild Type) qui est lié à au moins un espaceur PEGn qui est lui-même couplé à un azoture lié de façon covalente à un cyclooctyne DBCO qui est couplé à un peptide représenté par SEQ ID NO : 19 (β60-74_{cit60,72,74}). Fc-LALA-β60-74 représente un fragment Fc comprenant les mutations L234A, L235A et P329G qui est lié à au moins un espaceur PEGn qui est lui-même couplé à un azoture lié de façon covalente à un cyclooctyne DBCO qui est couplé à un peptide représenté par SEQ ID NO : 19. Fc-SDH-β60-74 représente un fragment Fc comprenant les mutations S239D, H268F, S324T et I332E qui est lié à au moins un espaceur PEGn qui est lui-même couplé à un azoture lié de façon covalente à un cyclooctyne DBCO couplé à un peptide représenté par SEQ ID NO : 19. Fc-WT-PEG-β60-74 représente un fragment Fc de type sauvage qui est lié à au moins un espaceur PEGn qui est lui-même couplé à un azoture lié de façon covalente à un cyclooctyne DBCO couplé à un espaceur PEGn qui est lui-même lié à un peptide représenté par SEQ ID NO : 19 (β60-74_{cit60,72,74}). Fc-SDH-PEG-β60-74 représente un fragment Fc comprenant les mutations S239D, H268F, S324T et I332E qui est lié à au moins un espaceur PEGn qui est lui-même couplé à un azoture lié de façon covalente à un cyclooctyne DBCO qui est couplé à un espaceur PEGn, lui-même lié à un peptide représenté par SEQ ID NO : 19 (β60-74_{cit60,72,74}). Fc-SDH(Cter)-PEG-β60-74 correspond au Fc-SDH-PEG-β60-74, à la différence près que dans l'hybride Fc-SDH(Cter)-PEG-β60-74, le premier PEGn est fixé au niveau de l'extrémité Cterminale du fragment Fc, contrairement aux autres exemples dans laquelle le premier PEGn est fixé au niveau de l'extrémité Nterminale. n représente un nombre entre 1 et 10 lorsqu'un espaceur PEGn est utilisé, de préférence 1, 2, 3, 4 ou 8.
**Fig. 5**
   [Fig. 5] **représente la réactivité d'une molécule hybride comprenant le peptide de SEQ ID NO** : **18 (α621-635cit_{621,627,630}) vis-à-vis des ACPA.**
   α621-635 représente le peptide de SEQ ID NO : 18. Fc-WT-PEG-α621-635 représente un fragment Fc de type sauvage (WT) qui est lié à au moins un espaceur PEGn qui est lui-même couplé à un azoture lié de façon covalente à un cyclooctyne DBCO couplé à un espaceur PEGn qui est lui-même lié à un peptide représenté par SEQ ID NO : 18. Fc-SDH-PEG-α621-635 représente un fragment Fc comprenant les mutations S239D, H268F, S324T et I332E qui est lié à au moins un espaceur PEGn qui est lui-même couplé à un azoture lié de façon covalente à un cyclooctyne DBCO qui est couplé à un espaceur PEGn qui est lui-même lié à un peptide représenté par SEQ ID NO : 18. n représente un nombre entre 1 et 10 lorsqu'un espaceur PEGn est utilisé, de préférence 1, 2, 3, 4 ou 8.
**Fig. 6**

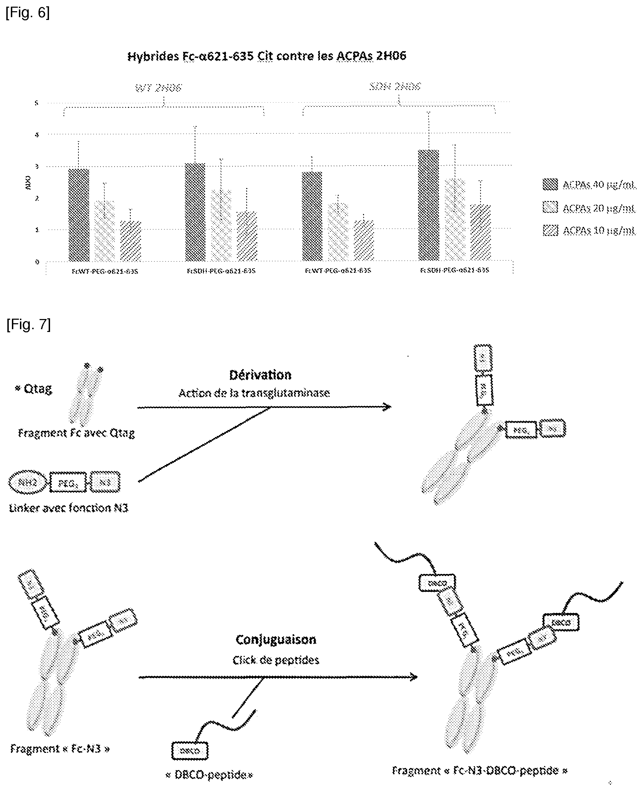

   [Fig. 6] **représente la réactivité d'un ACPA monoclonal humain recombinant (clone 2H06), comprenant un Fc non modifié ou modifié avec les mutations de type SDH, vis-à-vis des molécules hybrides comprenant le peptide de SEQ ID NO** : **18.**
   FcWT-PEG-α621-635 représente un fragment Fc de type sauvage qui est lié à au moins un espaceur PEGn qui est lui-même couplé à un azoture lié de façon covalente à un cyclooctyne DBCO couplé à un espaceur PEGn qui est lui-même lié à un peptide représenté par SEQ ID NO : 18. FcSDH-PEG-α621-635 représente un fragment Fc comprenant les mutations S239D, H268F, S324T et I332E, qui est lié à au moins un espaceur PEGn qui est lui-même couplé à un azoture lié de façon covalente à un cyclooctyne DBCO couplé à un espaceur PEGn qui est lui-même lié à un peptide représenté par SEQ ID NO : 18. n représente un nombre entre 1 et 10 lorsqu'un espaceur PEGn est utilisé, de préférence 1, 2, 3, 4 ou 8.
**Fig. 7**
   [Fig. 7] **représente un procédé de fabrication d'une molécule hybride selon l'invention, comprenant le schéma de dérivation et de conjugaison des fragments Fc.** **Fig. 8**
   [Fig. 8] **représente l'évolution du pourcentage de phagocytose et de lymphocytes B en présence de molécules hybrides Fc-WT-PEG-α621-635arg et Fc-WT-PEG-α621-635cit.** Les résultats des 6 expériences présentés en Exemple 6 sont indiqués (N=6). La valeur P est comme suit : * < 0,05 ** < 0,01 *** < 0,001.
**Fig. 9**
   [Fig. 9] **représente la lyse par des cellules NK humaines, de cellules d'une lignée cellulaire B humaine maligne lymphoblastique transduite exprimant à sa membrane un ACPA monoclonal recombinant, en présence des hybrides FcWT-N3-DBCO-PEG3-α621-635Cit et FcSDH-N3-DBCO-PEG3-α621-635Cit.**
   Nalm6 représente le cas où seules les cellules Nalm6 ont été incubées ; Nalm6+NK le cas où les cellules Nalm6 ont été incubées avec les cellules NK ; WTαCIT le cas où l'hybride WTαCIT a été incubé avec les cellules Nalm6 et les cellules NK ; WTαArg le cas où l'hybride WTαArg a été incubé avec les cellules Nalm6 et les cellules NK ; SDHαCIT le cas où l'hybride SDHαCIT a été incubé avec les cellules Nalm6 et les cellules NK et SDHαArg le cas où l'hybride SDHαArg a été incubé avec les cellules Nalm6 et les cellules NK.
   La lignée cellulaire B humaine maligne lymphoblastique transduite exprimant à sa membrane un ACPA monoclonal recombinant correspond à la lignée Nalm6 telle que décrite dans l'Exemple 11 et la Figure 10. L'anticorps monoclonal est le clone 022014CCP14CFCT2H06, dit 2H06.
**Fig. 10**
   [Fig. 10] **représente l'expression membranaire de l'ACPA monoclonal 2H06 par la lignée lymphoblastique B Nalm6 après transduction (Nalm6 2H06).**
   A- Analyse par cytométrie de flux de l'efficacité de transduction de la lignée B Nalm6 pour l'expression à sa membrane de l'ACPA monoclonal 2H06, grâce à un double marquage par un anticorps anti-Fab d'IgG et un tétramère du peptide α621-635cit (SEQ ID NO : 18). B- Analyse en cytométrie de flux de l'enrichissement en cellules transduites Nalm6 2H06, consécutif à un tri cellulaire réalisé grâce à un anticorps anti-Fab. C- Analyse en cytométrie de flux de l'interaction entre l'ACPA 2H06 membranaire exprimé par la lignée Nalm6 transduite et les hybrides Fc-WT-PEG-α621-635cit (cit) ou Fc-WT-PEG-α621-635arg (arg), couplés au fluorochrome A647. Nalm6 NT : lignée Nalm6 contrôle non transduite. Fc-WT-PEG-α621-635 représente un fragment Fc de type sauvage (WT) qui est lié à au moins un espaceur PEGn qui est lui-même couplé à un azoture lié de façon covalente à un cyclooctyne DBCO couplé à un espaceur PEGn qui est lui-même lié à un peptide représenté par SEQ ID NO : 18. Fc-WT-PEG-α621-635arg représente un fragment Fc de type sauvage (WT) qui est lié à au moins un espaceur PEGn qui est lui-même couplé à un azoture lié de façon covalente à un cyclooctyne DBCO couplé à un espaceur PEGn qui est lui-même lié à un peptide représenté par SEQ ID NO : 18 dans laquelle les résidus X ne sont pas citrullinés (i.e. ce sont des résidus arginyls).
   Les cellules Nalm6 exprimant l'ACPA monoclonal 2H06 correspondent à la lignée transduite Nalm6 mentionnée dans les exemples.
**Fig. 11**

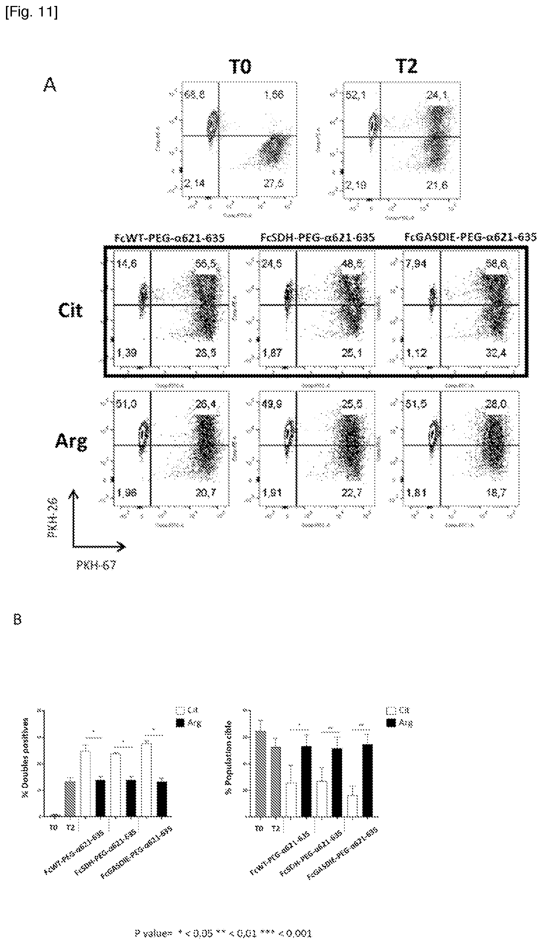

   [Fig. 11] **représente la phagocytose de cellules de la lignée B transduite Nalm6 ACPA+ 2H06 par des macrophages pré-armés d'hybrides Fc-peptide α621-635.**
   A- La Figure montre les résultats d'une expérience de cytométrie de flux où les populations cellulaires sont représentées sous forme de nuages de points. T0 représente le résultat au temps 0, après mise en contact des cellules-cibles Nalm6 et des macrophages. T2 représente le résultat au temps 2h, après 2h de mise en contact des cellules-cibles Nalm6 et des macrophages, en l'absence de molécules hybrides (phagocytose spontanée). L'ensemble des autres diagrammes de cytométrie représente les résultats obtenus après 2h de mise en contact des cellules-cibles Nalm6 et des macrophages armés d'hybrides Fc-PEG-α621-635cit (citrulliné) ou d'hybrides contrôles Fc-PEG-α621-635arg (non citrulliné). Les hybrides sont identiques à ceux décrits en Figure 10, à la différence près que différents types de fragment Fc ont été utilisés : un fragment Fc de type sauvage (WT), un fragment Fc GASDIE (présentant les mutations G236A, S239D et I332E), ou un fragment Fc SDH (présentant les mutations S239D, H268F, S324T et I332E). Les cellules présentant la double fluorescence (PKH-67 vert / PKH-26 rouge) correspondent aux macrophages qui ont phagocyté des cellules-cibles. Les cellules présentant une simple fluorescence PKH-26 correspondent aux cellules-cibles Nalm6 résiduelles, c'est-à-dire non phagocytées après 2h en présence des macrophages (% population-cible). Dans chaque cadran des diagrammes de cytométrie, les différentes populations cellulaires sont exprimées en pourcentage de la population cellulaire totale. B- Les Figures montrent sous forme d'histogrammes, pour chaque condition expérimentale décrite en A, la moyenne du % de cellules doubles-positives et la moyenne du % de cellules de la population-cible, calculées à partir des résultats de 3 expériences. Les histogrammes en noir représentent les conditions contrôles. Les histogrammes en blanc représentent les conditions en présence d'hybride Fc-PEG-α621-635cit. La valeur p est comme suit : * p< 0,05 ; ** p< 0,01.
**Fig. 12**

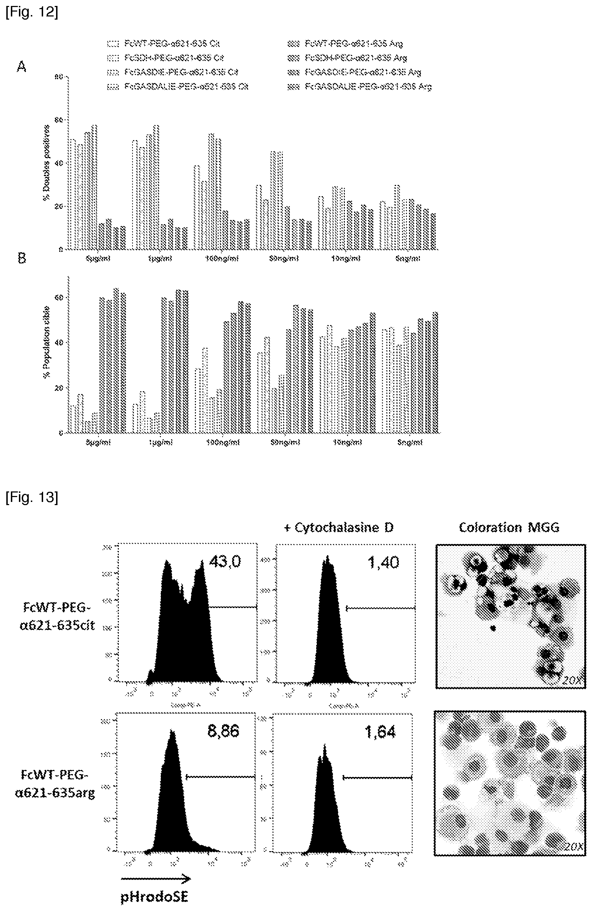

   [Fig. 12] **représente la phagocytose de cellules de la lignée B Nalm6 ACPA+ 2H06 par des macrophages pré-armés en présence d'hybrides Fc-peptide α621-635 à diverses concentrations.**
   La Figure montre les résultats d'une expérience de cytométrie de flux réalisée après phagocytose des cellules B Nalm6 2H06 par des macrophages pré-armés par incubation en présence d'hybrides Fc-peptide α621-635 à diverses concentrations. A- La Figure montre sous forme d'histogrammes, pour chaque condition expérimentale, le % de cellules doubles-positives. B-La Figure montre le % de la population Nalm6 cible résiduelle, c'est-à-dire non phagocytée. Les histogrammes sombres représentent les conditions contrôles obtenues en présence hybrides Fc-PEG-α621-635arg, non citrullinés. Les histogrammes clairs représentent les conditions en présence d'hybride Fc-PEG-α621-635cit. Les concentrations d'hybrides utilisées sont comprises entre 5 µg/ml et 5 ng/ml. Les hybrides sont identiques à ceux décrits en Figure 11, à la différence près qu'une molécule hybride avec un fragment Fc GASDALIE (comprenant les mutations G236A, S239D, A330L et I332E) a également été testée.
**Fig. 13**
   [Fig. 13] **représente la confirmation du processus de phagocytose de la lignée transduite Nalm6 ACPA+ 2H06 par des macrophages pré-armés d'hybrides FcWT-PEG-peptide α621-635.**
   Ces Figures montrent que les cellules doubles-positives observées en cytométrie dans les expériences précédentes correspondent bien à la phagocytose des cellules-cibles Nalm6 par les macrophages. Les quatre panneaux de gauche montrent des résultats de cytométrie de flux obtenus après mise en présence de cellules Nalm6 2H06 marquées au pHRodo SE et de macrophages armés d'hybrides Fc-WT-PEG-α621-635, ceci en présence ou non de cytochalasine D, inhibiteur de phagocytose. Les deux panneaux de droite montrent les populations cellulaires au terme de l'expérience, observées en microscopie optique après coloration au MGG. Les hybrides sont identiques à ceux décrits en Figure 10.
**Fig. 14**

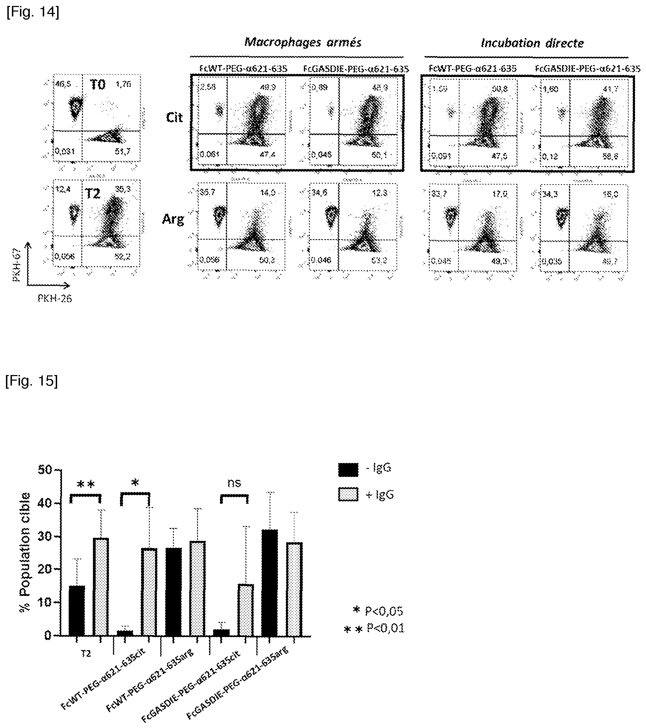

   [Fig. 14] **représente la phagocytose de la lignée transduite Nalm6 ACPA+ 2H06 par des macrophages pré-armés d'hybrides Fc-peptide α621-635 ou incubés en présence d'hybrides Fc-peptide α621-635.**
   La Figure montre les résultats d'une expérience analysée en cytométrie de flux : les nuages de points correspondent à des populations cellulaires. T0 représente le résultat au temps 0, juste après mise en contact des cellules-cibles Nalm6 et des macrophages. T2 représente le résultat à la 2ème heure après mise en contact des cellules-cibles Nalm6 et des macrophages, en l'absence de molécules hybrides (phagocytose spontanée). Tous les autres diagrammes de cytométrie présentent les résultats obtenus après 2h de mise en contact des cellules-cibles Nalm6 et des macrophages, soit pré-armés par les hybrides FcWT-PEG-α621-635 ou FcGASDIE-PEG-α621-635, citrullinés ou non, soit incubés avec les hybrides WT-PEG-α621-635 ou FcGASDIE-PEG-α621-635, citrullinés ou non, solubilisés dans le milieu d'incubation. Les hybrides sont identiques à ceux décrits en Figure 11.
   Les cellules présentant la double fluorescence (PKH-67 vert/PKH-26 rouge) correspondent aux macrophages qui ont phagocyté des cellules B cibles Nalm6 (% doubles positives). Les cellules présentant une simple fluorescence PKH-67 correspondent aux cellules B cibles Nalm6 résiduelles, c'est-à-dire non phagocytées après 2h en présence des macrophages. Dans chaque cadran des diagrammes de cytométrie, les différentes populations cellulaires sont exprimées en pourcentage de la population cellulaire totale.
**Fig. 15**
   [Fig. 15] **représente la phagocytose de la lignée transduite Nalm6 ACPA+ 2H06 par des macrophages incubés en présence des hybrides Fc-PEG-peptide α621-635 et, ou non, en présence d'IgG humaines à concentration sérique physiologique.**
   La Figure montre les moyennes des résultats de quatre (n=4) expériences identiques indépendantes, de phagocytose de cellules de la lignée transduite Nalm6 ACPA+ 2H06 par des macrophages, incubés en présence d'hybrides Fc-PEG-peptide α621-635, et en présence, ou non, d'IgG humaines à concentration sérique physiologique. La Figure montre pour chaque condition expérimentale, le pourcentage résiduel moyen de la population de cellules-cibles 2h après mise au contact de macrophages et d'hybrides Fc-PEG-α621-635 citrulliné (cit) ou non citrulliné (arg), solubles à la concentration d'1µg/mL, ceci en présence (histogrammes gris) ou en absence (histogrammes noirs), des IgG humaines compétitrices. La valeur de « p » est comme suit: * p < 0,05 ; ** p < 0,01. Les hybrides sont identiques à ceux décrits en Figure 11.
**Fig. 16**

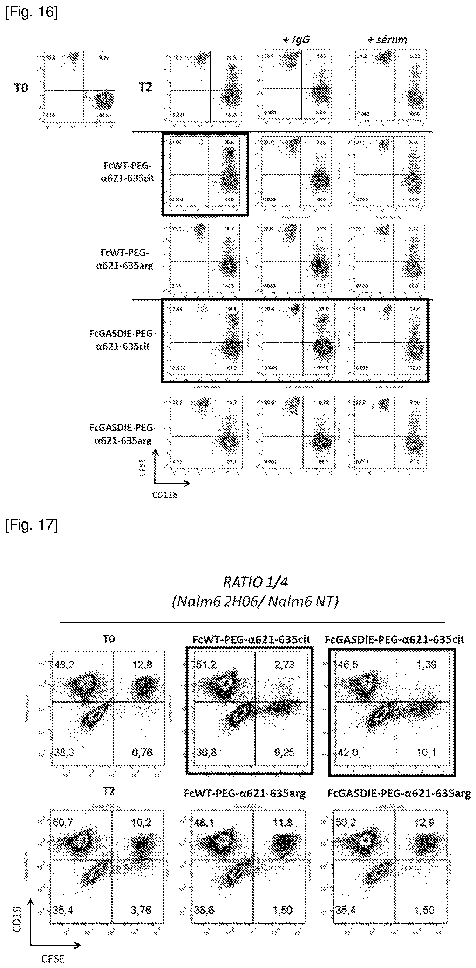

   [Fig. 16] **représente la phagocytose de la lignée transduite Nalm6 ACPA+ 2H06 par des macrophages humains incubés avec des hybrides Fc-peptide α621-635, en présence, ou non, d'IgG humaines à concentration physiologique ou de sérum humain.**
   La Figure montre les résultats d'une expérience analysée en cytométrie de flux où les populations cellulaires sont représentées par des nuages de points. T0 correspond au résultat obtenu immédiatement après mise en contact des cellules-cibles Nalm6 2H06 et des macrophages. T2 représente le résultat 2h après mise en contact des cellules-cibles et des macrophages en l'absence de molécules hybrides (phagocytose spontanée). Les autres diagrammes représentent les résultats obtenus 2h après mise en contact des cellules-cibles et des macrophages, incubés avec les hybrides FcWT-PEG-α621-635 ou FcGASDIE-PEG-α621-635, citrullinés (cit) ou non (arg), et ceci en présence ou non d'IgG humaines à la concentration sérique physiologique ou de sérum humain. Les cellules doubles-positives CFSE+ CD11b+ correspondent aux macrophages qui ont phagocyté des cellules-cibles. Les cellules simples-positives CFSE+ correspondent aux cellules-cibles Nalm6 résiduelles, non phagocytées après 2h. Dans chaque cadran des diagrammes de cytométrie, les différentes populations cellulaires sont exprimées en pourcentage de la population cellulaire totale. Les hybrides sont identiques à ceux décrits en Figure 11.
**Fig. 17**
   [Fig. 17] **représente la phagocytose de la lignée transduite Nalm6 ACPA+ 2H06 par des macrophages humains pré-armés avec les hybrides Fc-peptide α621-635, en présence de la lignée Nalm6 non transduite.**
   La Figure montre les résultats d'une expérience de phagocytose de cellules de la lignée transduite Nalm6 ACPA+ 2H06 par des macrophages pré-armés avec les hybrides Fc-peptide α621-635, en présence de cellules de la même lignée Nalm6, sauvages, non transduites. Les résultats de l'analyse en cytométrie de flux apparaissent sous la forme de nuages de points représentant les populations cellulaires. T0 représente la situation au temps 0, juste après mise en contact des cellules-cibles Nalm6 2H06, des cellules Nalm6 NT non transduites et des macrophages. T2 représente le résultat après 2h en l'absence de molécules hybrides (phagocytose basale spontanée). Tous les autres diagrammes de cytométrie représentent les résultats obtenus après 2h en présence des macrophages armés par les hybrides FcWT-PEG-α621-635 ou FcGASDIE-PEG-α621-635, citrullinés (cit) ou non citrullinés (arg). Les cellules doubles-positives CFSE+ CD19+ correspondent aux cellules-cibles Nalm6 2H06 résiduelles c'est-à-dire non phagocytées après 2h en présence des macrophages. Les cellules doubles-négatives CFSE- CD19- correspondent aux macrophages. Les cellules simples-positives CFSE- CD19+ correspondent aux cellules Nalm6 non-transduites (NT) résiduelles, c'est-à-dire non phagocytées après 2h en présence des macrophages. Les cellules simples positives CFSE+ CD19-correspondent aux macrophages qui ont phagocyté des cellules-cibles Nalm6 2H06. Dans chaque cadran des diagrammes de cytométrie, les différentes populations cellulaires sont exprimées en pourcentage de la population cellulaire totale. Les hybrides sont identiques à ceux décrits en Figure 11.
**Fig. 18**

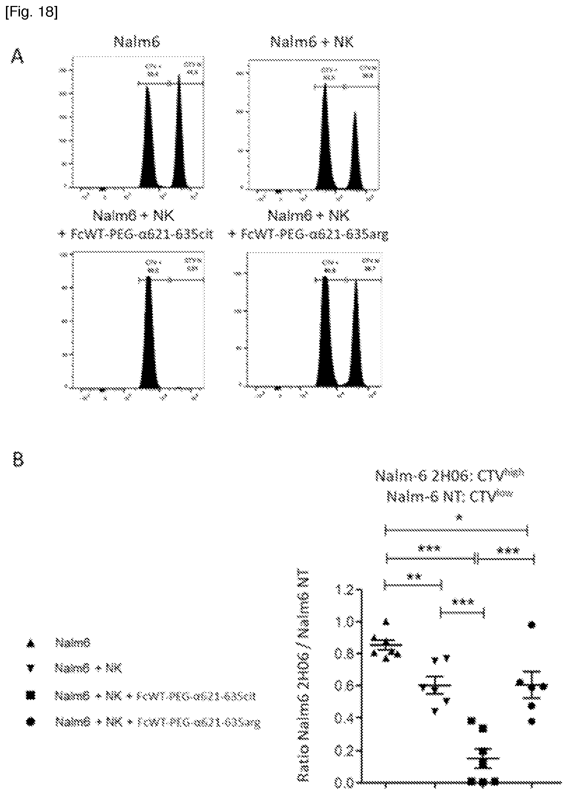

   [Fig. 18] **représente la destruction spécifique *in vivo* de la lignée Nalm6 ACPA+ 2H06 par des cellules NK humaines en présence d'hybrides Fc-peptide α621-635, chez des souris immunodéficientes SCID/BEIGE.**
   La Figure montre la destruction par des cellules NK humaines, dans la cavité péritonéale de souris immunodéficientes SCID/BEIGE, de cellules-cibles ACPA+ (lignée Nalm6 transduite 2H06) en présence d'hybrides Fc-peptide α621-635 citrullinés. A- La Figure représente l'analyse des populations de cellules Nalm6 2H06 et Nalm6 NT non-transduites, en cytométrie de flux. Les pics observés correspondent aux cellules-cibles (Nalm6 2H06 CTV^{high}) ou aux cellules contrôles (Nalm6 NT CTV^{low}). B- La figure représente l'ensemble des résultats obtenus pour quatre groupes de 7 souris immunodéficientes SCID/BEIGE. Ces résultats sont exprimés sous forme de ratio des fluorescences CTV^{high}/CTV^{low}, correspondant respectivement aux cellules Nalm6 2H06 (CTV^{high}) et aux cellules Nalm6 NT (CTV^{low}). Une diminution du ratio traduit la disparition des cellules-cibles Nalm6 2H06. La valeur « p » est comme suit : * p < 0,05 ; ** p < 0,01, *** p < 0,001. Les hybrides sont identiques à ceux décrits en Figure 10.
**Fig. 19**

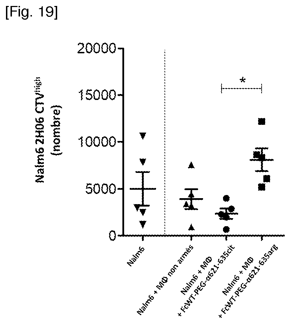

   [Fig. 19] **représente la destruction spécifique *in vivo* de la lignée Nalm6 ACPA+ 2H06 par des macrophages humains pré-armés avec les hybrides Fc-peptide α621-635, chez des souris immunodéficientes SCID/BEIGE.**
   La Figure montre la destruction par des macrophages humains, dans la cavité péritonéale de souris immunodéficientes SCID/BEIGE, de cellules-cibles ACPA+ (lignée Nalm6 transduite 2H06) en présence d'hybrides Fc-peptide α621-635 citrullinés. La Figure représente les résultats obtenus pour 4 groupes de 5 souris immunodéficientes SCID/BEIGE. Le nombre de cellules-cibles Nalm6 2H06 CTV^{high} résiduelles pour chaque groupe au terme de l'expérience est présenté, il indique l'efficacité de la phagocytose. La valeur « p » est comme suit : * p < 0,05. Les hybrides sont identiques à ceux décrits en Figure 10.

### Exemples

### Exemple 1 : Exemple de production d'une molécule hybride selon l'invention

La molécule hybride ici décrite comprend la construction suivante : un fragment Fc lié de façon covalente à au moins un espaceur PEGn, lui-même couplé à un azoture, ledit azoture étant lié de façon covalente à un alcyne, qui est lui-même couplé à un espaceur PEGn lié à un peptide citrulliné. Une telle molécule peut se lire : Fc-PEGn-N₃-DBCO-PEGn-peptide citrulliné. Les deux PEGn peuvent être identiques ou bien différents (par exemple le premier PEGn est PEG₃ et le deuxième PEGn est PEG₂).
1. Synthèse d'un NH2-PEGn-N₃ (i.e. un espaceur couplé à un azoture à une extrémité et possédant une fonction amine libre à une autre extrémité).
2. Mise en présence du NH₂-PEGn-N₃, avec un fragment Fc possédant un Qtag, et une transglutaminase, de préférence pendant 16h à 37°C. Cette étape dite « de dérivation » est de préférence réalisée avec 20 fois plus de moles de NH₂-PEGn-N₃ que de moles de fragment Fc. La transglutaminase est utilisée, à hauteur de 15U/µmol par Qtag présent (sur un ou sur les deux monomères). Eventuellement un dessalage peut être réalisé pour retirer l'excédent d'espaceur non lié au fragment Fc à l'issue de l'étape. Un Fc-PEGn-N₃ est ainsi obtenu. Si le fragment Fc comporte 2Qtag (un porté sur chaque monomère), alors le fragment Fc peut porter deux PEGn-N₃.
3. Synthèse d'un Cys-PEGn-peptide citrulliné (i.e. un espaceur lié à un peptide à une extrémité et possédant une cystéine libre à une autre extrémité). Un alcyne, par exemple un DBCO est ensuite couplé au Cys-PEGn-peptide citrulliné au niveau de la cystéine, et un DBCO-PEGn-peptide citrulliné est ainsi obtenu.
4. Réalisation du « click » : couplage entre le N₃ et le DBCO. Le DBCO-PEGn-peptide citrulliné est mis en présence du Fc-PEGn-N₃ avec, de préférence, 10 fois plus de moles de DBCO-PEGn-peptide citrulliné que de moles de Fc-PEGn-N₃. La réaction du click se déroule notamment à température ambiante et est presque complète au bout de 4h.
5. Obtention de la molécule hybride : Fc-PEGn-N₃-DBCO-PEGn-peptide citrulliné. Ce mode de réalisation est illustré en Figure 7.

De façon similaire un Fc-PEGn-DBCO et un N₃-PEGn-peptide citrulliné peuvent être obtenus, puis couplés pour obtenir Fc-PEGn-DBCO-N₃-PEGn-peptide citrulliné.

De façon similaire un Fc-PEGn-DBCO et un N₃-peptide citrulliné peuvent être obtenus, ou un Fc-PEGn-N₃ et un DBCO-peptide citrulliné, puis couplés pour obtenir respectivement Fc-PEGn-DBCO-N₃-peptide citrulliné ou Fc-PEGn-N₃-DBCO-peptide citrulliné.

### Exemple 2 : Réactivité des ACPA purifiés par chromatographie d'affinité sur peptides citrullinés et élués à pH3 (fraction pH3), vis-à-vis des molécules hybrides selon l'invention

Les ACPA (ici ACPA, fraction pH3) utilisés sont des ACPA obtenus à partir de sérum de patients atteints de polyarthrite rhumatoïde ACPA-positifs. De tels ACPA ont été obtenus par une méthode classique de chromatographie d'affinité sur colonne connue de l'homme de l'art, utilisant comme antigènes liés un ou plusieurs des cinq peptides immunodominants de l'invention (cf SEQ ID NO : 5, SEQ ID NO : 6, SEQ ID NO : 14, SEQ ID NO : 18 ou SEQ ID N O : 19).

### a. Exemples avec des molécules hybrides contenant le peptide β60-74cit

Des puits de plaque de microtitration ELISA ont été revêtus par adsorption passive à l'aide de 100 µL d'une solution contenant soit le peptide β60-74cit (SEQ ID NO : 19, également dénommé β60-74), soit une molécule hybride Fc-WT-β60-74cit, Fc-LALA-β60-74cit, Fc-SDH-β60-74cit, Fc-WT-PEG-β60-74cit, Fc-SDH-PEG-β60-74cit ou Fc-SDH(Cter)-PEG-β60-74cit. Ces solutions ont été utilisées chacune à la concentration de 5 µg/mL en tampon PBS (Phosphate Buffered Saline) et incubées pendant une nuit à 4°C. Le peptide non citrulliné et les molécules hybrides construites avec le peptide non citrulliné (β60-74arg, correspondant à un peptide de SEQ ID NO : 19 dans lequel les résidus citrullyl ont été remplacés par des résidus arginyl) ont été utilisés à la même concentration comme contrôles négatifs. Les puits ont ensuite été saturés par la BSA (Bovine Serum Albumin) à 2%, pendant 1h à 4°C. Après lavages, les ACPA purifiés ont été incubés à 1, 0,5 ou 0,25 µg/mL, dilués en tampon PBS 2% BSA 2M NaCl pendant 1h à 4°C. Après lavages, la réactivité des ACPA a été détectée à l'aide d'un anticorps secondaire anti-Fab d'IgG humaines dilué au 1/2500 en tampon PBS 2% BSA. Les résultats sont exprimés en ΔDO (Delta-Densité Optique), correspondant à la DO obtenue avec le peptide β60-74cit ou avec les molécules hybrides construites avec le peptide β60-74cit, soustraite respectivement de la DO obtenue avec le peptide β60-74arg ou avec les molécules hybrides correspondantes construites avec le peptide β60-74arg.

Les résultats sont présentés en Figure 4. Ils montrent une réactivité dose-dépendante des ACPA purifiés sur peptide β60-74cit, vis-à-vis des molécules hybrides Fc-WT-β60-74cit, Fc-LALA-β60-74cit, Fc-SDH-β60-74cit, Fc-WT-PEG-β60-74cit, Fc-SDH-PEG-β60-74cit ou Fc-SDH(Cter)-PEG-β60-74cit. Ils montrent qu'inclus dans les différents hybrides, les peptides citrullinés restent parfaitement réactifs avec les ACPA.

### b. Exemples avec des molécules hybrides contenant le peptide α621-635cit

Des puits de plaque ELISA ont été revêtus à l'aide de 100 µL d'une solution contenant le peptide α621-635cit (SEQ ID NO : 18, également dénommé α621-635) ou une molécule hybride Fc-WT-PEG-α621-635cit ou encore Fc-SDH-PEG-α621-635cit, à la concentration de 5 µg/mL en tampon PBS (Phosphate Buffered Saline), incubée pendant une nuit à 4°C. Le peptide non citrulliné α621-635arg et les molécules hybrides correspondantes construites avec le peptide non citrulliné (α621-635arg, correspondant à un peptide de SEQ ID NO : 18 dans lequel les résidus citrullyl ont été remplacés par des résidus arginyl) ont été utilisés à la même concentration comme contrôles négatifs. Une saturation des puits a ensuite été réalisée par la BSA (Bovine Serum Albumin) à 2% en tampon PBS pendant 1h à 4°C. Après lavages, les ACPA purifiés ont été incubés à 4, 2 et 1 µg/mL dilués en tampon PBS 2% BSA 2M NaCl pendant 1h à 4°C. Après lavages, la réactivité des ACPA a été détectée à l'aide d'un anticorps secondaire anti-Fab d'IgG humaines dilué au 1/2500 en tampon PBS 2% BSA. Les résultats sont exprimés en ΔDO (Delta-Densité Optique), correspondant à la DO obtenue avec le peptide *α* 621-635cit ou avec les molécules hybrides construites avec le peptide α621-635cit, soustraite respectivement de la DO obtenue avec le peptide α621-635arg ou avec les molécules hybrides construites avec le peptide α621-635arg.

Les résultats sont présentés en Figure 5. Ils montrent une forte réactivité dose-dépendante des ACPA purifiés sur peptide α621-635cit vis-à-vis des molécules hybrides Fc-WT-PEG-α621-635cit et Fc-SDH-PEG-α621-635cit. La réactivité des ACPA vis-à-vis des molécules hybrides est comparativement plus forte, alors que la densité épitopique qu'elles présentent est inférieure à celle du peptide α621-635cit.

### Exemple 3 : Réactivité des ACPA monoclonaux recombinants 2H06 sous forme sauvage (wild type - WT) ou mutée sur leur fragment Fc (mutation SDH), vis-à-vis des molécules hybrides selon l'invention

L'ACPA ici utilisé est un ACPA humain monoclonal recombinant ((clone 022014CCP14CFCT2H06, dit 2H06). Un tel ACPA peut être obtenu comme décrit dans Titcombe P. J., Wigerblad G., Sippl N., Zhang N., Shmagel A. K., Sahiström P., Zhang Y., Barsness L. O., Ghodke - Puranik Y., Baharpoor A., et al. Pathogenic Citrulline-Multispecific B Cell Receptor Clades in Rheumatoid Arthritis. Arthritis & Rheumatology 2018, 70 (12), 1933-1945. https://doi.org/10.1002/art.40590. Le VH de l'ACPA 2H06 a le numéro d'accession MH629710.1 sous Genbank, et le VL a le numéro d'accession MH629700.1.

Des puits de plaque ELISA ont été revêtus à l'aide de 100 µL d'une solution contenant soit la molécule hybride Fc-WT-PEG- *α* 621-635cit, soit Fc-SDH-PEG- *α* 621-635cit, utilisée à la concentration de 5 µg/mL en tampon PBS (Phosphate Buffered Saline) incubées pendant une nuit à 4°C. Les molécules hybrides construites avec un peptide non citrulliné ( *α* 621-635arg) ont été utilisées à la même concentration comme contrôles négatifs. Une saturation des puits a ensuite été réalisée en tampon PBS 2% BSA (Bovine Serum Albumin), pendant 1h à 4°C. Après lavages, les ACPA monoclonaux recombinants 2H06, WT et SDH, ont été incubés à 40, 20 et 10 µg/mL, dilués en tampon PBS 2% BSA 2M NaCl pendant 1h à 4°C. Après lavages, leurs réactivités ont été détectées à l'aide d'un anticorps secondaire anti-Fab d'IgG humaines dilué au 1/2500 en tampon PBS 2% BSA. Les résultats sont exprimés en ΔDO (Delta-Densité Optique), correspondant à la DO obtenue avec les molécules hybrides construites avec le peptide *α* 621-635cit soustraite de la DO obtenue avec les molécules hybrides construites avec le peptide contrôle négatif *α* 621-635arg.

Les molécules hybrides Fc-WT-PEG-α621-635cit et Fc-SDH-PEG-α621-635cit sont identiques à celles utilisées dans l'Exemple 2.

Les résultats sont présentés en Figure 6. Les résultats montrent une forte réactivité dose-dépendante, des ACPA monoclonaux WT et SDH vis-à-vis des molécules hybrides Fc-WT-PEG-α621-635cit et Fc-SDH-PEG-α621-635cit.

### Exemple 4 : Fixation des molécules hybrides sur macrophages à 2h et 20h, à température physiologique

Des macrophages humains, différenciés *in vitro* en présence de M-CSF (100ng/mL) à partir de monocytes CD14+ isolés du sang périphérique d'un sujet sain ont été incubés à 500.000 cellules/puits, en présence de molécules hybrides à 5 µg/mL, FcWT-N3-DBCO-β60-74Cit (un fragment Fc de type sauvage qui est lié à un espaceur PEGn qui est lui-même couplé à un azoture lié de façon covalente à un cyclooctyne DBCO qui est couplé à un peptide représenté par SEQ ID NO : 19, dénommé WT dans les Tableaux 2 et 3), FcSDHFSTIE-N3-DBCO-β60-74Cit (un fragment Fc comprenant les mutations S239D, H268F, S324T et I332E qui est lié à un espaceur PEGn qui est lui-même couplé à un azoture lié de façon covalente à un DBCO qui est couplé à un peptide représenté par SEQ ID NO : 19, dénommé SDH dans les Tableaux 2 et 3) ou FcLALAPG-N3-DBCO-β60-74Cit (un fragment Fc comprenant les mutations L234A, L235A et P329G qui est lié à un espaceur PEGn qui est lui-même couplé à un azoture lié de façon covalente à un cyclooctyne DBCO qui est couplé à un peptide représenté par SEQ ID NO : 19, dénommé LALAPG dans les Tableaux 2 et 3), pendant 2h ou 20h à 37°C. La fixation des molécules hybrides à la surface des macrophages est mise en évidence et quantifiée par cytofluorimétrie (FACS Canto II) après incubation des macrophages avec un anticorps anti-Fc couplé au FITC, utilisé 1/1000. Dans les constructions, n représente un nombre entre 1 et 10 lorsqu'un espaceur PEGn est utilisé, de préférence 1, 2, 3, 4 ou 8.

Les résultats sont présentés dans les Tableaux 2 et 3 ci-après. « NM » et « ANTI FC » représentent respectivement le puits contenant les macrophages incubés sans anticorps ou avec l'anticorps anti-Fc seul.

**[Tableau 2]. Résultats à 2h**

| 2h | Macrophages | Ratio |
|---|---|---|
| NM | 177 | / |
| ANTI FC | 285 | / |
| WT | 808 | 2.8 |
| LALAPG | 502 | 1.8 |
| SDH | 1330 | 4.7 |

**[Tableau 3]. Résultats à 20h**

| 20h | Macrophages | Ratio |
|---|---|---|
| NM | 213 | / |
| ANTI FC | 275 | / |
| WT | 1003 | 3.6 |
| LALAPG | 404 | 1.5 |
| SDH | 1457 | 5.3 |

Les résultats présentés dans les Tableaux 2 et 3, montrent que les molécules hybrides FcWT-N3-DBCO-β60-74Cit et FcSDHFSTIE-N3-DBCO-β60-74Cit à la température physiologique de 37°C, se fixent dès la 2^{ème} heure à la membrane des macrophages et y sont encore présentes 20h plus tard. Ces résultats montrent également que la fixation de l'hybride FcSDHFSTIE-N3-DBCO-β60-74Cit est supérieure à celle de la forme sauvage FcWT-N3-DBCO-β60-74Cit.

### Exemple 5 : Phagocytose induite par l'hybride Fc-SDH peptide β60citrulliné lorsqu'on arme les lymphocytes B via leurs FcγRIIb (CD32b) avec les ACPA purifiés

Des lymphocytes B humains fraîchement purifiés à partir de sang périphérique de donneurs sains par tri magnétique CD19+, ont été marqués au PKH-67 vert (Paul Karl Horan / marqueur lipidique fluorescent) puis incubés avec des ACPA purifiés (fraction pH3, obtenus comme dans l'Exemple 2) sur peptide β60-74cit (SEQ ID NO : 19) à une concentration de 5 µg/mL durant 30min à 37°C. Par la suite, les cellules ont été lavées puis mises en présence de l'hybride FcSDHFSTIE-N3-DBCO-PEG3-β60-74Cit (un fragment Fc comprenant les mutations S239D, H268F, S324T et I332E qui est lié à au moins un espaceur PEGn qui est lui-même couplé à un azoture lié de façon covalente à un cyclooctyne DBCO qui est couplé à un espaceur PEGn lui-même lié à un peptide représenté par SEQ ID NO : 19). Cette construction est dénommée CIT dans le Tableau 4 ci-après). Les cellules ont également été mises en présence de l'hybride FcSDHFSTIE-N3-DBCO-PEG-β60-74Arg (qui correspond à la construction FcSDHFSTIE-N3-DBCO-PEG3-β60-74Cit dans laquelle le peptide β60-74 n'est pas citrulliné). Cette construction est dénommée NCIT dans le Tableau 4 ci-après). Les cellules ont été mises en présence des hybrides à 10 µg/mL durant 30 min à 37°C. Après lavage, les cellules ont été mises au contact des macrophages (marqués PKH-26 rouge), à raison de 1 lymphocyte B pour 1 macrophage, durant 2h à 37°C. Les cellules ont ensuite été décollées puis analysées par cytométrie en flux. Les cellules présentant la double fluorescence (PKH-67 vert/PKH-26 rouge) correspondent aux macrophages qui ont phagocyté des lymphocytes. Les différentes populations cellulaires sont exprimées en pourcentage de la population cellulaire totale : pourcentage de phagocytose et pourcentage de lymphocytes B. Dans les constructions, n représente un nombre entre 1 et 10 lorsqu'un espaceur PEGn est utilisé, de préférence 1, 2, 3, 4 ou 8.

Les résultats sont présentés dans le Tableau 4 selon quatre lignes qui correspondent à quatre expériences indépendantes.

**[Tableau 4]. Pourcentage de phagocytose et pourcentage de lymphocytes B**

| % Phagocytose | | | % Population B | | |
|---|---|---|---|---|---|
| NCIT | CIT | % (ΔCIT-NCIT) | NCIT | CIT | % (ΔCIT-NCIT) |
| 9.28 | 17.8 | +91% | 46.1 | 32.9 | - 40% |
| 16.2 | 20.4 | +26% | 40.5 | 30.5 | - 33% |
| 3.42 | 5 | +46% | 77.1 | 66.7 | - 16% |
| 6.28 | 8.95 | +42% | 77.3 | 69.3 | - 12% |

Les résultats obtenus confirment que l'activité de phagocytose est majorée en présence des hybrides citrullinés (augmentation de la phagocytose associée à une diminution de la population de lymphocytes B). En effet, dans les 4 expériences indépendantes, une augmentation significative du pourcentage de phagocytose, toujours associée à une diminution significative de la population de lymphocytes B, a été observée lorsque les lymphocytes B étaient recouverts de l'hybride FcSDHFSTIE-N3-DBCO-PEG3-β60-74Cit (CIT) versus FcSDHFSTIE-N3-DBCO-PEG3-β60-74Arg (NCIT).

### Exemple 6 : Phagocytose de lymphocytes B chargés avec l'ACPA humain monoclonal recombinant 2H06 muté SDH, par des macrophages armés avec l'hybride Fc-WT-PEG-α621-635cit

Des lymphocytes B humains fraîchement purifiés à partir de sang périphérique de donneurs sains par tri magnétique CD19+, ont été marqués au PKH-67 vert (Paul Karl Horan / marqueur lipidique fluorescent) puis incubés durant 1h à 37°C avec l'ACPA monoclonal humain recombinant 2H06 dont le Fc est muté SDH, à une concentration de 10 µg/mL. De tels anticorps peuvent être obtenus comme dans l'Exemple 3. En parallèle, des macrophages marqués au PKH-26 rouge ont été mis en présence des hybrides Fc-WT-PEG-α621-635cit (un fragment Fc de type sauvage qui est lié à au moins un espaceur PEGn qui est lui-même couplé à un azoture lié de façon covalente à un DBCO qui est couplé à un espaceur PEGn qui est lui-même lié au peptide représenté par SEQ ID NO : 18) et Fc-WT-PEG-α621-635arg (il s'agit de l'hybride Fc-WT-PEG-α621-635cit, à la différence près que le peptide n'est pas citrulliné), à 5 µg/mL durant 1 h à 37°C. Après lavage, les cellules ont été mises en contact, à raison de 1 lymphocyte B pour 1 macrophage, durant 2h à 37°C. Les cellules ont ensuite été décollées puis analysées par cytométrie en flux. Le pourcentage de phagocytose correspond au pourcentage de cellules marquées par les 2 fluorochromes (PKH-67 vert/PKH-26 rouge). Dans les constructions, n représente un nombre entre 1 et 10 lorsqu'un espaceur PEGn est utilisé, de préférence 1, 2, 3, 4 ou 8.

Les résultats sont présentés dans le Tableau 5 selon six lignes qui correspondent à cinq expériences indépendantes. NCIT représente le résultat avec le peptide Fc-WT-PEG-α621-635arg, CIT représente le résultat avec le peptide Fc-WT-PEG-α621-635cit, et (Δ CIT-NCIT) représente la différence entre la DO obtenue en présence de Fc-WT-PEG-α621-635cit et la DO obtenue en présence de Fc-WT-PEG-α621-635arg, divisée par la DO obtenue en présence de Fc-WT-PEG-α621-635arg.

**[Tableau 5]. Pourcentage de phagocytose et pourcentage de lymphocytes B**

| % Phagocytose | | |
|---|---|---|
| NCIT | CIT | % (ΔCIT-NCIT) |
| 4.3 | 7.0 | +63% |
| 15.8 | 19.2 | +21% |
| 4.6 | 7.3 | +58% |
| 12.8 | 18.2 | +42% |
| 16.9 | 21.4 | + 27% |
| 15,7 | 20.1 | + 28% |

| % Population B | | |
|---|---|---|
| NCIT | CIT | % (ΔCIT-NCIT) |
| 66.6 | 61.2 | - 8% |
| 78.7 | 76.6 | - 3% |
| 67.6 | 57.2 | - 15% |
| 54.2 | 47.7 | - 12% |
| 44.5 | 37 | - 17% |
| 43.8 | 38.5 | - 12% |

Les résultats montrent que l'activité de phagocytose est majorée en présence des hybrides citrullinés : augmentation de la phagocytose (de 21 à 63%), associée à une diminution de la population de lymphocytes B (de 3 à 17%). Dans chacune des six expériences, le % de doubles-positifs est plus élevé, donc la phagocytose est majorée et la population B diminue lorsque les macrophages sont armés avec les hybrides Fc-WT-PEG-α621-635cit (CIT) versus Fc-WT-PEG-α621-635arg (NCIT). Les résultats sont également présentés en Figure 8A et 8B.

### Exemple 7 : Stabilité de la fixation des Fc-SDH A488 et des hybrides Fc-SDH peptide β60 citrulliné, sur des cellules NK après 30min, 24h et 48h, à température physiologique

Les cellules NK ont été incubées 30min, 24h ou 48h à 37°C en présence de FcSDHFSTIE-N3-DBCO-A488 (un fragment Fc comprenant les mutations S239D, H268F, S324T et I332E qui est lié à au moins un espaceur PEGn qui est lui-même couplé à un azoture lié de façon covalente à un DBCO, la molécule étant marquée avec le fluorochrome A488) ou de FcSDHFSTIE-N3-DBCO-PEG3-β60-74cit-lysineA488 (un fragment Fc comprenant les mutations S239D, H268F, S324T et I332E qui est lié à au moins un espaceur PEGn qui est lui-même couplé à un azoture lié de façon covalente à un DBCO qui est couplé à espaceur PEGn lié à un peptide de SEQ ID NO : 19, la molécule étant marquée avec le fluorochrome A488 qui est liée à la molécule via une lysine). La fixation de ces 2 sondes fluorescentes aux cellules NK a été analysée par cytofluorimétrie. Dans les constructions, n représente un nombre entre 1 et 10 lorsqu'un espaceur PEGn est utilisé, de préférence 1, 2, 3, 4 ou 8.

Les résultats sont présentés dans les Tableaux 6 (30 minutes), 7 (24 heures) et 8 (48 heures) suivants. NM représente les cellules NK non marquées, sans anticorps.

**[Tableau 6]. Résultats à 30 minutes**

| | MFI | RATIO |
|---|---|---|
| NM | 562 | / |
| FcSDHFSTIE-N3-DBCO-PEG3-β60-74cit-lysineA488 | 4588 | 8,163 |
| FcSDHFSTIE-N3-DBCO-A488 | 2973 | 5,289 |

**[Tableau 7]. Résultats à 24 heures**

| | MFI | RATIO |
|---|---|---|
| NM | 558 | / |
| FcSDHFSTIE-N3-DBCO-PEG3-β60-74cit-lysineA488 | 4091 | 7,324 |
| FcSDHFSTIE-N3-DBCO-A488 | 2353 | 4,212 |

**[Tableau 8]. Résultats à 48 heures**

| | MFI | RATIO |
|---|---|---|
| NM | 494 | / |
| FcSDHFSTIE-N3-DBCO-PEG3-β60-74cit-lysineA488 | 4336 | 8,77 |
| FcSDHFSTIE-N3-DBCO-A488 | 1710 | 3,460 |

Les résultats présentés dans les Tableaux 6 à 8, montrent que le FcSDHFSTIE-N3-DBCO-A488 et le FcSDHFSTIE-N3-DBCO-PEG3-β60-74cit-lysine A488 restent fixés à la membrane des cellules NK au moins jusqu'à 48h à température physiologique et que la fixation du peptide sur le fragment Fc n'empêche pas son interaction avec les Fc gamma récepteurs des cellules NK.

### Exemple 8 : Fixation sur cellules NK des diverses formes de Fc (WT, SDH et LALAPG) marquées par le fluorochrome A647

Les cellules NK ont été incubées 30min à 37°C soit en présence du fragment FcSDHFSTIE-N3-DBCO-A647 (un fragment Fc comprenant les mutations S239D, H268F, S324T et I332E qui est lié à au moins un espaceur PEGn qui est lui-même couplé à un azoture lié de façon covalente à un DBCO, la molécule étant marquée avec le fluorochrome A647) à 10 µg/mL. Cette construction est dénommée SDH dans le Tableau 9 ci-après. L'expérience a également été réalisée avec les hybrides suivants : FcWT-N3-DBCO-A647 (un fragment Fc de type sauvage qui est lié à au moins un espaceur PEGn qui est lui-même couplé à un azoture lié de façon covalente à un DBCO, la molécule étant marquée avec le fluorochrome A647), ou FcLALAPG-N3-DBCO-A647 (un fragment Fc comprenant les mutations L234A, L235A et P329G qui est lié à au moins un espaceur PEGn qui est lui-même couplé à un azoture lié de façon covalente à un DBCO, la molécule étant marquée avec le fluorochrome A647), aux mêmes concentrations. Ces deux constructions sont respectivement dénommées WT et LALAPG dans le Tableau 9 ci-après. Dans les constructions, n représente un nombre entre 1 et 10 lorsqu'un espaceur PEGn est utilisé, de préférence 1, 2, 3, 4 ou 8. L'analyse de la fixation des fragments Fc aux cellules NK a été réalisée par cytofluorométrie. Les résultats sont présentés dans le Tableau 9.

**[Tableau 9]. Résultats**

| | MFI | RATIO |
|---|---|---|
| NEG | 212.08 | / |
| WT 10 µg/mL | 1105.89 | 5.21 |
| SDH 10 µg/mL | 38545.45 | 181.74 |
| LALAPG 10 µg/mL | 450.43 | 2.12 |

Les résultats présentés dans le Tableau 9, montrent que les fragments FcWT-N3-DBCO-A647, FcSDHFSTIE-N3-DBCO-A647 se fixent à la membrane des cellules NK. La fixation du FcSDHFSTIE-N3-DBCO-A647 est beaucoup plus importante que celle du FcWT-N3-DBCO-A647.

### Exemple 9 : Fixation sur cellules NK du fragment Fc-SDH marqué par le fluorochrome A647 comparée à la fixation des mêmes hybrides en présence d'IgG humaines à des concentrations croissantes, jusqu'à la concentration sérique physiologique

Les cellules NK ont été incubées 30min à 37°C soit en présence du fragment FcSDHFSTIE-N3-DBCO-A647 (un fragment Fc comprenant les mutations S239D, H268F, S324T et I332E qui est lié à au moins un espaceur PEGn qui est lui-même couplé à un azoture lié de façon covalente à un DBCO, la molécule étant marquée avec le fluorochrome A647) à 10 µg/mL, soit en présence de ce même fragment FcSDHFSTIE-N3-DBCO-A647 à 10 µg/mL, co-incubé avec des IgG humaines à concentrations croissantes (1, 10, 100, 1.000, 10.000 µg/mL). Dans les constructions, n représente un nombre entre 1 et 10 lorsqu'un espaceur PEGn est utilisé, de préférence 1, 2, 3, 4 ou 8. La fixation du fragment FcSDHFSTIE-N3-DBCO-A647 (dit SDH) aux cellules NK a été analysée par cytofluorométrie.

Les résultats sont présentés dans le Tableau 10.

**[Tableau 10]. Résultats**

| | MFI | RATIO |
|---|---|---|
| NEG | 212.08 | / |
| SDH 10 µg/mL | 38545.45 | 181.74 |
| SDH (1µg/mL IgG) | 37899.98 | 178.70 |
| SDH (10µg/mL IgG) | 38771.43 | 182.81 |
| SDH (100µg/mL IgG) | 36270.21 | 171.02 |
| SDH (1000µg/mL IgG) | 29526.19 | 139.22 |
| SDH (10000µg/mL IgG) | 20486.26 | 96.59 |

Les résultats montrent, une diminution dose-dépendante de la fixation du FcSDHFSTIE-N3-DBCO-A647 aux cellules NK à partir de 100 µg/mL d'IgG mais celle-ci reste effective et élevée, même en présence d'une concentration en IgG 1.000 fois supérieure à celle du fragment Fc (10.000 µg/mL) qui correspond à la concentration sérique physiologique des IgG chez l'Homme.

### Exemple 10 : Lyse par des cellules NK humaines, de cellules d'une lignée B humaine maligne plasmoblastique exprimant à sa membrane un ACPA monoclonal recombinant, en présence des hybrides FcWT-N3-DBCO-PEG3-α621-635Cit et FcSDH-N3-DBCO-PEG3-α621-635Cit

WTαCIT représente l'hybride comprenant un fragment Fc de type sauvage qui est lié à au moins un espaceur PEGn qui est lui-même couplé à un azoture lié de façon covalente à un cyclooctyne DBCO qui est couplé à un espaceur PEGn qui est lui-même lié au peptide représenté par SEQ ID NO : 18 ; WTαArg correspond à l'hybride WTαCIT à la différence près que le peptide de SEQ ID NO : 18 n'est pas citrulliné ; SDHαCIT représente un fragment Fc comprenant les mutations S239D, H268F, S324T et I332E qui est lié à au moins un espaceur PEGn qui est lui-même couplé à un azoture lié de façon covalente à un cyclooctyne DBCO qui est couplé à un espaceur PEGn qui est lui-même lié au peptide représenté par SEQ ID NO : 18 ; et SDHαArg correspond à l'hybride SDHαCIT à la différence près que le peptide de SEQ ID NO : 18 n'est pas citrulliné. Dans les constructions, n représente un nombre entre 1 et 10 lorsqu'un espaceur PEGn est utilisé, de préférence 1, 2, 3, 4 ou 8.

L'expérience a été réalisée en plaques de 96 puits à fond rond. Des cellules de la lignée Nalm6 (transduite pour l'expression membranaire de l'ACPA monoclonal humain recombinant 2H06) ont été utilisées comme cellules-cibles. 100.000 cellules/puits ont été incubées en présence, d'une part, des molécules hybrides WTαCit ou SDHαCit utilisées à 15µg/mL et, d'autre part, des cellules NK fraichement purifiées (marquées avec le cell tracker violet) utilisées comme cellules effectrices, également à 100.000 cellules/puits, durant 17h à 37°C. Les molécules hybrides WTαArg et SDHαArg, utilisées comme contrôles de spécificité, ont été incubées dans les mêmes conditions. Comme contrôles négatifs, les cellules Nalm6 ont été incubées en l'absence d'hybrides, seules ou en présence de cellules NK. La lyse cellulaire a été mesurée en cytométrie de flux par un marquage avec la sonde fluorescente 7-AAD. Les résultats montrent le pourcentage de cellules positives pour ce marqueur (% de cellules mortes). (%) (ΔCIT-Arg) représente la différence entre les pourcentages de cellules mortes 7-AAD-positives en présence des hybrides porteurs de peptides citrullinés WT et SDH versus leurs homologues non-citrullinés, divisée par le pourcentage de cellules 7-AAD-positives en présence de l'hybride non citrulliné correspondant.

Les résultats sont présentés en Figure 9 et dans le Tableau 11.

**[Tableau 11]. Résultats**

| | *% 7-AAD* + | *% **(ΔCIT-Arg)*** |
|---|---|---|
| **Nalm6** | 8 | |
| **Nalm6 + NK** | 45 | |
| **Nalm6 + NK + WT αCIT** | 65 | ***+ 62 %*** |
| **Nalm6 + NK + WT αArg** | 40 | |
| **Nalm6 + NK + SDH αCIT** | 55 | ***+ 83 %*** |
| **Nalm6 + NK + SDH αArg** | 30 | |

Les résultats montrent que les hybrides citrullinés (WT et SDH) sont capables de majorer spécifiquement la lyse par ADCC des cellules-cibles Nalm6 ACPA-positives, provoquée par les cellules NK. Cette augmentation de mortalité est de 62 % pour les hybrides WT et de 83 %, pour les hybrides SDH.

### Exemple 11 : Expression membranaire de l'ACPA monoclonal 2H06 par la lignée lymphoblastique B Nalm6 après transduction

A- La lignée cellulaire humaine B lymphoblastique « Nalm6 » a été transduite pour induire l'expression à sa membrane de l'ACPA monoclonal recombinant 2H06. L'efficacité de transduction a été analysée en cytométrie de flux grâce à un double marquage utilisant un anticorps anti-Fab d'IgG et un tétramère du peptide α621-635cit biotinylé fixé Avidine fluorescente. B- Un tri réalisé à partir de 70 millions de cellules Nalm6 a permis de récupérer les cellules Nalm6 Fab+ dont un pool de 300.000 cellules a été remis en culture. Après prolifération, l'enrichissement de la lignée transduite Nalm6 2H06 a été confirmé par cytométrie de flux. C- Les hybrides Fc-WT-PEG-α621-635 cit et arg ont été marqués à l'Alexa Fluor 647 à l'aide du kit « Lightning Link ». Les lignées Nalm6 non transduite (NT) et Nalm6 2H06, ont été incubées avec les hybrides Fc-WT-PEG-α621-635. Après lavage, la fixation membranaire des hybrides Fc-WT-PEG-α621-635 a été analysée par cytométrie de flux.

Les résultats sont présentés dans la Figure 10 et dans le Tableau 12.

Le Tableau 12 représente les intensités moyennes de fluorescence (MFI) et le ratio de MFI entre la condition en présence d'hybride fluorescent et la condition contrôle où les cellules ne sont pas marquées. nm représente les cellules Nalm6 non marquées, sans hybrides fluorescents.

**[Tableau 12]. Résultats**

| | MFI - Nalm6 NT | Ratio - Nalm6 NT | MFI - Nalm6-2H06 | Ratio - Nalm6-2H06 |
|---|---|---|---|---|
| nm | 37 | | 37 | |
| cit | 263 | 7 | 10783 | 290 |
| arg | 260 | 7 | 263 | 7 |

Les résultats montrent qu'après tri et prolifération cellulaire, 89,1% des cellules Nalm6 expriment un Fab d'IgG, celui de l'ACPA 2H06, permettant de valider le succès de la transduction et l'établissement de la lignée Nalm6 2H06 (B). Les résultats montrent une interaction forte entre la lignée Nalm6 2H06 et l'hybride Fc-WT-PEG-α621-635 cit fluorescent, alors qu'aucune interaction n'existe avec l'hybride Fc-WT-PEG-α621-635 arg, non citrulliné (C). Un double marquage avec l'hybride Fc-WT-PEG-α621-635 cit fluorescent et l'anticorps anti-Fab d'IgG fluorescent, montre une parfaite corrélation des 2 marquages sur la lignée Nalm6 2H06, alors qu'aucun des deux ne marque la lignée Nalm6 non transduite (A). Ces résultats montrent que la fixation de l'hybride Fc-WT-PEG-α621-635 cit est strictement liée à l'expression de l'ACPA 2H06 membranaire.

### Exemple 12 : Phagocytose de cellules de la lignée B transduite Nalm6 ACPA+ 2H06 par des macrophages pré-armés d'hybrides Fc-peptide α621-635

Les macrophages ont été marqués avec le fluorochrome membranaire PKH-67 vert puis ont été incubés avec les hybrides Fc-WT-PEG-α621-635, Fc-SDH-PEG-α621-635 ou Fc-GASDIE-PEG-α621-635, sous forme citrullinée (Cit) ou non citrullinée (Arg), à une concentration de 5µg/ml durant 60 min à 37°C. Les cellules de la lignée humaine lymphoblastique B Nalm6 transduite pour l'expression de l'ACPA monoclonal 2H06 à sa membrane, ont été marquées au PKH-26 rouge (marqueur lipidique fluorescent) puis mises en présence des macrophages à raison de 2 cellules-cibles Nalm6 pour 1 macrophage. Après 2h à 37°C, les macrophages ont été décollés puis les populations cellulaires analysées par cytométrie en flux.

Les résultats de la Figure 11A montrent qu'à T2, en l'absence d'hybrides, il existe une augmentation des cellules doubles-positives qui témoigne d'une phagocytose basale spontanée des cellules Nalm6. A 2 heures, en présence d'hybrides, quel que soit l'hybride utilisé (WT, SDH ou GASDIE), une forte augmentation des cellules doubles-positives et un effondrement du nombre des cellules-cibles, sont observés lorsque les macrophages sont armés d'hybrides citrullinés, mais absents avec les versions non citrullinées des hybrides. On passe ici de 52% de cellules doubles-positives à 14,6% en présence de l'hybride Fc-WT-PEG-α621-635, 24,5% pour Fc-SDH-PEG-α621-635 et jusqu'à 8% pour Fc-GASDIE-PEG-α621-635 qui est le plus efficace.

La synthèse de 3 expériences indépendantes (voir Figure 11B), confirme que l'effet est reproductible et significatif dans toutes les conditions, avec une augmentation moyenne de 70% des cellules doubles-positives et une diminution d'au moins 50% des cellules-cibles Nalm6.

### Exemple 13 : Phagocytose de cellules de la lignée B Nalm6 ACPA+ 2H06 par des macrophages pré-armés en présence d'hybrides Fc-peptide α621-635 à diverses concentrations

Les macrophages ont été marqués avec le fluorochrome membranaire PKH-67 vert puis armés avec les hybrides Fc-WT-PEG-α621-635, Fc-SDH-PEG-α621-635 Fc-GASDIE-PEG-α621-635 ou Fc-GASDALIE-PEG-α621-635, sous forme citrullinée (Cit) ou non citrullinée (Arg), à différentes concentrations (5µg/ml ; 1µg/ml ; 100ng/ml ; 50ng/ml ; 10ng/ml ; 5ng/ml) durant 60 min à 37°C. Les cellules de la lignée B Nalm6 transduite pour l'expression de l'ACPA monoclonal 2H06, ont été marquées au PKH-26 rouge (marqueur lipidique fluorescent) puis mises en présence des macrophages armés, à raison d'1 cellule-cible Nalm6 pour 1 macrophage. Après 2h à 37°C, les macrophages ont été décollés puis les populations cellulaires analysées par cytométrie en flux.

Les résultats présentés dans la Figure 12 montrent une forte augmentation du pourcentage de cellules doubles-positives (A) associée à une forte diminution du pourcentage de cellules-cibles (B), lorsque les macrophages sont armés avec les hybrides Fc-PEG-α621-635 citrullinés, qu'ils soient WT, SDH, GASDIE ou GASDALIE. L'une et l'autre sont absentes avec les hybrides non citrullinés (Fc-PEG-α621-635 Arg). Cette phagocytose spécifique est corrélée avec la concentration des hybrides Fc-PEG-α621-635 dans la solution utilisée pour les armer. Le modèle est particulièrement robuste car la phagocytose est encore observée pour tous les hybrides citrullinés jusqu'à 50ng/mL. Du fait de leurs affinités accrues vis-à-vis des FcγR, les hybrides Fc-GASDIE-PEG-α621-635Cit et Fc-GASDALIE-PEG-α621-635Cit, sont plus efficaces que les Fc-WT-PEG-α621-635Cit ou Fc-SDH-PEG-α621-635Cit, et restent actifs au-delà de 50 ng/ml.

### Exemple 14 : Confirmation du processus de phagocytose de la lignée transduite Nalm6 ACPA+ 2H06 par des macrophages armés d'hybrides FcWT-PEG-peptide α621-635

Pour confirmer que la destruction des cellules B Nalm6 2H06 résulte d'un processus de phagocytose, des cellules B Nalm6 2H06 ont été marquées avec un colorant sensible au pH, le pHRodoSE, qui présente une faible intensité de fluorescence à pH neutre mais émet une fluorescence intense en milieu acide. Or le pH dans les vésicules intra-cytoplasmiques (lysosomes) où se retrouvent les éléments phagocytés est acide. Les cellules non phagocytées ne sont donc pas détectables, alors que les cellules phagocytées, internalisées dans le compartiment lysosomal, deviennent fluorescentes.

Ainsi, des cellules Nalm6 2H06 ont été marquées avec le pHRodoSE (Thermofisher) à 20ng/ml. Les cellules Nalm6 marquées ont été incubées avec les macrophages armés ou non d'hybrides Fc-WT-PEG-α621-635 Cit ou Arg, à 5µg/ml pendant 1h à 37°C, traités ou non par la cytochalasine D pendant 30 min à 2µg/ml puis 1µg/ml au cours de la phagocytose. Les cellules ont été incubées 2h à 37°C, puis les macrophages ont été décollés et les populations cellulaires analysées par cytométrie en flux ou colorées au May Grunwald Giemsa (MGG) pour analyse morphologique en microscopie optique.

Les résultats présentés dans la Figure 13 montrent l'apparition d'une population pHRodoSE-positive (2ème pic) en présence de macrophages armés de l'hybride Fc-WT-PEG-α621-635 Cit, population qui est absente lorsque les macrophages sont armés de la version non citrullinée des hybrides. En présence de cytochalasine D (inhibiteur de la phagocytose), cette population n'apparaît pas, confirmant qu'elle correspond bien à une population de macrophages ayant phagocytée spécifiquement des cellules B Nalm6 2H06. Ces résultats sont confirmés par une analyse en microscopie optique dans laquelle on observe de nombreuses cellules B Nalm6 2H06 phagocytées, en situation intra-cytoplasmique dans des macrophages, lorsqu'ils sont armés d'hybrides Fc-WT-PEG-α621-635 citrulliné (panneau haut), alors que cette situation est totalement absente lorsque les macrophages étaient armés de la forme non citrullinée de l'hybride.

### Exemple 15 : Phagocytose de la lignée transduite Nalm6 ACPA+ 2H06 par des macrophages pré-armés d'hybrides Fc-peptide α621-635 ou incubés en présence d'hybrides Fc-peptide α621-635

Les cellules de la lignée B Nalm6 2H06 ont été marquées par le fluorochrome membranaire PKH-67 vert et les macrophages par le PKH-26 rouge. Les hybrides Fc-WT-PEG-α621-635 et Fc-GASDIE-PEG-α621-635, sous forme citrullinée (Cit) ou non citrullinée (Arg), ont été soit incubés durant 1h à 37°C avec les macrophages à la concentration de 5µg/ml (armement suivi de lavage), avant leur mise en contact des cellules-cibles selon le ratio 1 cellule-cible Nalm6 pour 1 macrophage, pendant 2h à 37°C, soit incubés à la même concentration dans le milieu contenant les cellules-cibles Nalm6 et les macrophages selon le même ratio 1/1, pendant 2h à 37°C. Les macrophages ont été ensuite décollés et les populations cellulaires analysées par cytométrie de flux.

Les résultats présentés dans la Figure 14 montrent une forte augmentation des cellules doubles-positives (phagocytose), associée à un effondrement de la population de cellules-cibles Nalm6 2H06, lorsque les macrophages ont été soit pré-armés, soit incubés avec les hybrides Fc-WT-PEG-α621-635 ou Fc-GASDIE-PEG-α621-635 sous forme citrullinée. Ce résultat est spécifique puisque non retrouvé avec les formes non-citrullinées des hybrides.

### Exemple 16 : Phagocytose de la lignée transduite Nalm6 ACPA+ 2H06 par des macrophages incubés avec des hybrides Fc-PEG-peptide α621-635, en présence ou non d'IgG humaines à concentration sérique physiologique

Les cellules de la lignée Nalm6 2H06 ont été marquées avec le colorant fluorescent CFSE (CarboxyFluorescein Succinimidyl Ester) puis incubées avec des macrophages en présence d'hybrides Fc-WT-PEG-α621-635 ou Fc-GASDIE-PEG-α621-635, sous forme citrullinée (Cit) ou non citrullinée (Arg), à la concentration d'1µg/mL, pendant 2h à 37°C, en présence ou non, d'IgG humaines compétitrices à la concentration sérique physiologique de 10mg/mL (rapport de concentration entre hybrides et IgG : 1/10.000). Les macrophages ont été décollés puis marqués avec un anticorps anti-CD11b BV421 et les populations cellulaires ont été analysées en cytométrie de flux.

Les résultats présentés dans la Figure 15 montrent qu'en absence d'IgG compétitrices, les formes citrullinées des 2 hybrides solubles Fc-WT-PEG-α621-635 et Fc-GASDIE-PEG-α621-635 incubés avec les cellules, induisent une phagocytose quasi-totale et spécifique car absente avec les formes non-citrullinées, de la population de cellules Nalm6 2H06. La présence des IgG induit une inhibition relative mais significative (p<0.01) de la phagocytose spontanée à T2, une inhibition quasi-totale et significative de la phagocytose induite par l'hybride Fc-WT-PEG-α621-635cit (p<0.05), alors que cette inhibition est n'est que partielle et non significative avec l'hybride Fc-GASDIE-PEG-α621-635, dont le Fc, muté, est plus affin pour les Fc gamma récepteurs macrophagiques que celui des IgG sériques.

### Exemple 17 : Phagocytose de la lignée transduite Nalm6 ACPA+ 2H06 par des macrophages humains incubés avec des hybrides Fc-peptide α621-635, en présence ou non d'IgG humaines à concentration physiologique ou de sérum humain

Les cellules de la lignée Nalm6 2H06 ont été marquées avec le colorant fluorescent CFSE (CarboxyFluorescein Succinimidyl Ester) puis incubées avec des macrophages en présence d'hybrides Fc-WT-PEG-α621-635 ou Fc-GASDIE-PEG-α621-635 sous forme citrullinée (cit) ou non citrullinée (arg), à la concentration de 10µg/mL. L'expérience s'est déroulée pendant 2h à 37°C en présence ou non d'IgG humaines compétitrices à la concentration de 10mg/mL ou d'un mélange de sérums humains, présentant la même concentration en IgG. Le rapport de concentration entre hybrides et IgG est donc ici de 1/1000. Les cellules ont été prélevées et marquées avec un anticorps anti-CD11 b BV421 afin d'identifier les macrophages puis analysées en cytométrie de flux.

Les résultats présentés dans la Figure 16 montrent que les 2 hybrides Fc-WT-PEG-α621-635 et Fc-GASDIE-PEG-α621-635, citrullinés, utilisés à la concentration de 10µg/ml, induisent spécifiquement et très efficacement la phagocytose des cellules Nalm6 2H06. Par compétition avec les hybrides, les IgG inhibent la phagocytose des cellules Nalm6 2H06 par les macrophages mais l'effet inhibiteur est beaucoup plus fort pour l'hybride Fc-WT (sauvage) que pour l'hybride GASDIE (muté, plus affin pour les Fc gamma Récepteurs macrophagiques). Des résultats équivalents sont obtenus en présence de sérum humain montrant que celui-ci ne contient donc pas d'autres facteurs inhibiteurs de la phagocytose que les IgG.

### Exemple 18 : Phagocytose de la lignée transduite Nalm6 ACPA+ 2H06 par des macrophages humains pré-armés avec les hybrides Fc-peptide α621-635, en présence de la lignée Nalm6 non transduite

Les cellules de la lignée Nalm6 2H06 ont été marquées avec le colorant fluorescent CFSE (CarboxyFluorescein Succinimidyl Ester) puis mélangées à des cellules Nalm6 NT non transduites selon le ratio ¼ (1 cellule transduite pour 4 cellules non-transduites). Les macrophages ont été pré-armés avec les hybrides Fc-WT-PEG-α621-635 ou Fc-GASDIE-PEG-α621-635, sous forme citrullinée (Cit) ou non citrullinée (Arg), par une incubation d'1h à la concentration d'1g/mL à 37°C. Les cellules Nalm6 ont ensuite été mises en présence des macrophages pendant 2h à 37°C, puis les macrophages ont été décollés, la population cellulaire totale prélevée et marquée avec un anticorps anti-CD19 APC, afin de discriminer cellules Nalm6 2H06 et cellules Nalm6 NT non-transduites. La population cellulaire totale a été ensuite analysée en cytométrie de flux.

Pour confirmer la spécificité de la phagocytose à l'égard des cellules ACPA+ en présence des hybrides citrullinés, nous avons co-incubé les cellules-cibles Nalm6 2H06 (ACPA+) et les cellules Nalm6 NT non transduites (ACPA-). Les résultats présentés dans la Figure 17 montrent que seules les cellules Nalm6 exprimant l'ACPA membranaire 2H06 (CFSE+CD19+) disparaissent et se retrouvent phagocytées par les macrophages lorsque ceux-ci ont été préalablement armés avec les hybrides Fc-WT-PEG-α621-635 ou Fc-GASDIE-PEG-α621-635 sous forme citrullinée (Cit). Au contraire, la proportion de cellules Nalm6 NT, sauvages non transduites ACPA- (CD19+CFSE-) est inchangée, quelles que soient les conditions, confirmant que l'interaction induite est bien spécifique et due à la liaison du peptide citrulliné et des ACPA. Les mêmes résultats ont été obtenus pour un ratio de 1/9 (1 cellule Nalm6 2H06 pour 9 cellules Nalm6 NT non transduites).

### Exemple 19 : Destruction spécifique in vivo de la lignée Nalm6 ACPA+ 2H06 par des cellules NK humaines en présence d'hybrides Fc-peptide α621-635, chez des souris immunodéficientes SCID/BEIGE

L'expression membranaire des ACPA 2H06 sur les cellules-cibles Nalm6 2H06 a été contrôlée par marquage avec un anticorps anti-Fab et l'activité lytique (ADCC) des cellules NK humaines effectrices vérifiée *in vitro.* Les cellules-cibles Nalm6 2H06 ont été marquées avec le marqueur fluorescent CTV (CellTrace Violet) à forte concentration (5µM) alors que des cellules Nalm6 NT non transduites, ont été marquées avec le même marqueur mais à une concentration dix fois inférieure (0,5µM). En cytométrie de flux, les cellules fortement marquées (Nalm6 2H06 CTV^{high}) peuvent ainsi être distinguées des cellules faiblement marquées (Nalm6 NT CTV^{low}). Les souris immunodéficientes SCID/BEIGE ont reçu 50µg d'anticorps anti-FcR en intra-péritonéal (IP) afin de bloquer les FcR des macrophages péritonéaux murins puis,30 minutes plus tard, les cellules-cibles Nalm6 2H06 et des cellules Nalm6 NT non transduites, ont été injectées en IP en présence, ou non, de cellules NK humaines fraîchement préparées et des hybrides Fc-WT-PEG-α621-635 citrullinés (cit) ou non (arg).

Quatre groupes de 7 souris SCID/BEIGE ont reçu en IP les combinaisons de cellules et d'hybrides suivantes :
- 1/ 2.10⁵ cellules-cibles Nalm6-2H06 + 2.10⁵ cellules Nalm6 NT
- 2/ 2.10⁵ cellules-cibles Nalm6-2H06 + 2.10⁵ cellules Nalm6 NT + 2.10⁵ cellules NK
- 3/ 2.10⁵ cellules-cibles Nalm6-2H06 + 2.10⁵ cellules Nalm6 NT + 2.10⁵ cellules NK + 3µg/souris d'hybride Fc-WT-PEG-α621-635 Cit
- 4/ 2.10⁵ cellules-cibles Nalm6-2H06 + 2.10⁵ cellules Nalm6 NT + 2.10⁵ cellules NK + 3µg/souris d'hybride Fc-WT-PEG-α621-635 Arg

Cinq heures après l'injection, les cellules ont été récupérées par lavage péritonéal avec du PBS 2% SVF puis analysées par cytométrie de flux après marquage avec 7AAD et Annexine (kit Becton Dickinson).

Les résultats présentés dans la Figure 18 montrent que dans la cavité péritonéale des souris immunodéficientes SCID/BEIGE, l'hybride Fc-WT-PEG-α621-635 Cit induit la destruction totale et spécifique des cellules-cibles Nalm6 2H06 par les cellules NK. En effet, le pic CTV^{high} correspondant aux cellules Nalm6 2H06 disparait exclusivement en présence des cellules NK et de l'hybride Fc-WT-PEG-α621-635 Cit.

Ce résultat très significatif (***, p<0.001) apporte la preuve *in vivo* de l'efficacité et de la spécificité de la lyse par ADCC des cellules ACPA+ par les cellules NK humaines en présence d'hybrides Fc-peptides citrullinés porteurs des épitopes-cibles des ACPA.

### Exemple 20 : Destruction spécifique in vivo de la lignée Nalm6 ACPA+ 2H06 par des macrophages humains pré-armés avec les hybrides Fc-peptide α621-635, chez des souris immunodéficientes SCID/BEIGE

L'expression membranaire des ACPA 2H06 sur les cellules-cibles Nalm6 2H06 a été contrôlée par marquage avec un anticorps anti-Fab. La purification des monocytes a été effectuée à partir de sang périphérique d'un donneur sain. La différenciation des monocytes en macrophages a été induite en présence de 100 ng/ml de M-CSF et l'activité phagocytaire des macrophages ainsi générés a été vérifiée *in vitro.*

Avant injection intra-péritonéale (IP), les macrophages ont été armés avec les différents hybrides Fc-WT-PEG-α621-635 citrullinés (cit) ou non (arg) et les cellules-cibles Nalm6-2H06 ont été marquées avec le marqueur fluorescent CTV (CellTrace Violet) à 0,5µM : fortement fluorescentes lorsqu'elles sont libres (Nalm6 2H06 CTV^{high}), ces cellules présentent une fluorescence beaucoup plus faible (Nalm6-2H06 CTV^{low}) après phagocytose par les macrophages.

Quatre groupes de 5 souris SCID/BEIGE ont reçu en IP les cellules-cibles, en présence, ou non, de macrophages frais pré-armés et, ou non, d'hybrides FcWT-PEG-α621-635 cit ou arg, selon les combinaisons suivantes :
- 1/ 2.10⁵ cellules-cibles Nalm6-2H06
- 2/ 2.10⁵ cellules-cibles Nalm6-2H06 + 2.10⁵ macrophages non armés
- 3/ 2.10⁵ cellules-cibles Nalm6-2H06 + 2.10⁵ macrophages pré-armés d'hybride Fc-WT-PEG-α621-635 Cit
- 4/ 2.10⁵ cellules cibles Nalm6-2H06 2.10⁵ macrophages pré-armés d'hybride Fc-WT-PEG-α621-635 Arg

Deux heures après l'injection, les cellules ont été récupérées par lavage péritonéal avec du PBS 5% SVF, les érythrocytes ont été éliminés par une lyse douce et les cellules ont été marquées avec un anticorps anti-CD45 humain-FITC puis avec du 7AAD (kit Becton Dickinson) avant analyse en cytométrie de flux.

Les résultats présentés dans la Figure 19 montrent que dans la cavité péritonéale des souris immunodéficientes SCID/BEIGE, des macrophages chargés par l'hybride Fc-WT-PEG-α621-635 cit phagocytent spécifiquement des cellules-cibles Nalm6 2H06, leur diminution par rapport au contrôle (hybride Fc-WT-PEG-α621-635 arg, non citrulliné) étant significative (p<0.05).

Ce résultat apporte la preuve *in vivo* de l'efficacité et de la spécificité de la phagocytose des cellules ACPA+ par des macrophages humains chargés d'hybrides Fc-peptides citrullinés porteurs des épitopes-cibles des ACPA.

## Revendications

1. Molécule hybride comprenant au moins un fragment Fc d'anticorps lié de façon covalente à au moins un peptide dérivé de fibrine ayant au moins un résidu citrullyl, au moins un espaceur étant optionnellement présent entre ledit fragment Fc et ledit peptide, ledit peptide étant reconnu par les auto-anticorps anti-protéines citrullinées et étant dérivé de tout ou partie de la séquence de la chaine α ou β d'une fibrine de vertébré, par substitution d'au moins un résidu arginyl par un résidu citrullyl.

2. Molécule hybride selon la revendication précédente, ladite fibrine de vertébré étantune fibrine de mammifère, plus particulièrement humaine.

3. Molécule hybride selon l'une quelconque des revendications précédentes, dans laquelle ledit espaceur est un polymère contenant un ou plusieurs motifs de répétition contenant le groupe éther, ledit espaceur étant de préférence le polyéthylène glycol de formule PEGn, dans laquelle n représente un nombre entier compris entre 1 et 100, préférentiellement entre 1 et 10 et notamment 1, 2, 3, 4 ou 8.

4. Molécule hybride selon l'une quelconque des revendications précédentes, dans laquelle ledit peptide comprend au moins un résidu citrullyl, et est choisi dans le groupe constitué par :
a) un peptide défini par la séquence X₁PAPPPISGGGYX₂AX₃ (SEQ ID NO : 1) dans laquelle X₁, X₂, et X₃ représentent chacun un résidu citrullyl ou un résidu arginyl, et au moins l'un des résidus X₁ ou X₂ ou X₃ est un résidu citrullyl ;
b) un peptide défini par la séquence GPX₁VVEX₂HQSACKDS (SEQ ID NO : 2) dans laquelle X₁ et X₂ représentent chacun un résidu citrullyl ou un résidu arginyl, et au moins l'un des résidus X₁ ou X₂ est un résidu citrullyl ;
c) un peptide défini par la séquence SGIGTLDGFX₁HX₂HPD (SEQ ID NO : 3) dans laquelle X₁ et X₂ représentent chacun un résidu citrullyl ou un résidu arginyl, et au moins l'un des résidus X₁ ou X₂ est un résidu citrullyl ;
d) un peptide défini par la séquence VDIDIKIX₁SCX₂GSCS (SEQ ID NO : 4) dans laquelle X₁ et X₂ représentent chacun un résidu citrullyl ou un résidu arginyl, et au moins l'un des résidus X₁ ou X₂ est un résidu citrullyl ;
e) un peptide défini par la séquence X₁GHAKSX₂PVX₃GIHTS (SEQ ID NO : 12) dans laquelle X₁, X₂ et X₃ représentent chacun un résidu citrullyl ou un résidu arginyl, et au moins l'un des résidus X₁ ou X₂ ou X₃ est un résidu citrullyl ;
f) un peptide comprenant au moins 5 acides aminés consécutifs, dont au moins un résidu citrullyl, de l'un des peptides a) à e) ci-dessus.

5. Molécule hybride selon l'une quelconque des revendications 1-3, dans laquelle ledit peptide est choisi dans le groupe constitué par : SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22 et SEQ ID NO : 23, plus particulièrement choisi dans le groupe constitué par : SEQ ID NO : 5, SEQ ID NO : 6, SEQ ID NO : 14, SEQ ID NO : 18 et SEQ ID NO : 19.

6. Molécule hybride selon l'une quelconque des revendications précédentes, dans laquelle ledit fragment Fc est un fragment Fc humain, notamment d'IgG, plus particulièrement d'IgG1.

7. Molécule hybride selon l'une quelconque des revendications précédentes, dans laquelle ledit fragment Fc est de type sauvage ou muté, ledit fragment Fc muté comprenant de préférence au moins les mutations suivantes :
- L234A et L235A, ou
- L234A, L235A et P329G, ou
- G236A, S239D et I332E, ou
- G236A, S239D, A330L et I332E, ou
- S239D, H268F, S324T et I332E,
la numérotation étant indiquée dans la séquence d'une IgG1 humaine selon l'index EU.

8. Molécule hybride selon l'une quelconque des revendications précédentes, dans laquelle :
- le fragment Fc est couplé à au moins un azoture et ledit peptide est couplé à un alcyne, tel qu'un cyclooctyne, et en particulier le DBCO, ou
- le fragment Fc est couplé à au moins un alcyne, tel qu'un cyclooctyne, et en particulier le DBCO, et ledit peptide est couplé à un azoture, ou
- le fragment Fc est couplé à au moins un azoture et ledit peptide est lié à un espaceur lui-même couplé à un alcyne, tel qu'un cyclooctyne, et en particulier le DBCO ou
- le fragment Fc est couplé à au moins un alcyne, tel qu'un cyclooctyne, et en particulier le DBCO, et ledit peptide est lié à un espaceur lui-même couplé à un azoture ou
- le fragment Fc est lié à au moins un espaceur, lui-même couplé à un azoture, et ledit peptide est couplé à un alcyne, tel qu'un cyclooctyne, et en particulier le DBCO ou
- le fragment Fc est lié à au moins un espaceur, lui-même couplé à un alcyne, tel qu'un cyclooctyne, et en particulier le DBCO, et ledit peptide est couplé à un azoture ou
- le fragment Fc est lié à au moins un espaceur, lui-même couplé à un azoture, et ledit peptide est lié à un espaceur, lui-même couplé à un alcyne, tel qu'un cyclooctyne, et en particulier le DBCO ou
- le fragment Fc est lié à au moins un espaceur, lui-même couplé à un alcyne, tel qu'un cyclooctyne, et en particulier le DBCO, et ledit peptide est lié à un espaceur, lui-même couplé à un azoture,
la liaison covalente entre ledit fragment Fc et ledit peptide, éventuellement en présence d'un ou plusieurs espaceurs, étant créée entre l'azoture et l'alcyne.

9. Molécule hybride selon l'une quelconque des revendications précédentes, ladite molécule étant représentée par :
- un fragment Fc de type sauvage qui est lié à au moins un espaceur PEGn qui est lui-même couplé à un azoture lié de façon covalente à un cyclooctyne qui est couplé à un peptide représenté par SEQ ID NO : 19 ou SEQ ID NO : 18,
- un fragment Fc comprenant les mutations S239D, H268F, S324T et I332E qui est lié à au moins un espaceur PEGn qui est lui-même couplé à un azoture lié de façon covalente à un cyclooctyne qui est couplé à un peptide représenté par SEQ ID NO : 19 ou SEQ ID NO : 18,
- un fragment Fc comprenant les mutations L234A, L235A et P329G qui est lié à au moins un espaceur PEGn qui est lui-même couplé à un azoture lié de façon covalente à un cyclooctyne qui est couplé à un peptide représenté par SEQ ID NO : 19 ou SEQ ID NO : 18,
- un fragment Fc de type sauvage qui est lié à un espaceur PEGn qui est lui-même couplé à au moins un azoture lié de façon covalente à un cyclooctyne couplé à un espaceur PEGn qui est lui-même lié à un peptide représenté par SEQ ID NO : 19 ou SEQ ID NO : 18,
- un fragment Fc comprenant les mutations S239D, H268F, S324T et I332E qui est lié à au moins un espaceur PEGn qui est lui-même couplé à un azoture lié de façon covalente à un cyclooctyne couplé à un espaceur PEGn qui est lui-même lié à un peptide représenté par SEQ ID NO : 19 ou SEQ ID NO : 18,
n représentant de préférence un entier entre 1 et 10, plus particulièrement 1, 2, 3 ou 4.

10. Molécule hybride selon l'une quelconque des revendications précédentes, pour son utilisation comme médicament, notamment pour son utilisation dans le traitement des maladies auto-immunes associées à la production d'auto-anticorps anti-protéines citrullinées, en particulier le syndrome de Gougerot-Sjögren et la Polyarthrite Rhumatoïde.

11. Molécule hybride selon l'une quelconque des revendications précédentes, pour son utilisation comme médicament, en combinaison avec une deuxième molécule hybride, ladite deuxième molécule hybride comprenant au moins un peptide dérivé de fibrine ayant au moins un résidu citrullyl, ledit peptide étant lié de façon covalente à au moins un anticorps ou à au moins un fragment d'anticorps, ledit anticorps ou fragment étant capable de se lier à CD38 et/ou CD138, un ou plusieurs espaceurs étant optionnellement présents entre ledit peptide et ledit anticorps ou ledit fragment.

## Patentansprüche

1. Hybridmolekül, umfassend mindestens ein Fc-Fragment eines Antikörpers, das kovalent an mindestens ein aus Fibrin abgeleitetes Peptid, das mindestens einen Citrullinrest aufweist, gebunden ist, wobei gegebenenfalls zwischen dem Fc-Fragment und dem Peptid mindestens ein Spacer vorliegt, wobei das Peptid von Anti-citrulliniertes Protein-Auto-Antikörpern erkannt wird und von der gesamten oder einem Teil der Sequenz der α- oder β-Kette eines Vertebratenfibrins durch Substitution mindestens eines Arginylrests duch einen Citrulinrest abgeleitet ist.

2. Hybridmolekül nach dem vorangehenden Anspruch, wobei das Vertebratenfibrin Fibrin eines Säugers ist, insbesondere humanes Fibrin.

3. Hybridmolekül nach einem der vorangehenden Ansprüche, wobei der Spacer ein Polymer ist, das ein oder mehrere Wiederholungseinheiten enthält, die eine Ethergruppe enthält/enthalten, wobei der Spacer bevorzugt Polyethylenglycol der Formel PEGn ist, wobei n eine ganze Zahl zwischen 1 und 100, bevorzugt zwischen 1 und 10, und insbesondere 1, 2, 3, 4 oder 8 ist.

4. Hybridmolekül nach einem der vorangehenden Ansprüche, wobei das Peptid mindestens einen Citrullinrest umfasst und ausgewählt ist aus der Gruppe bestehend aus:
a) einem Peptid, das durch die Sequenz X₁PAPPPISGGGYX₂AX₃ (SEQ ID NO:1) definiert ist, wobei X₁, X₂ und X₃ jeweils einen Citrullinrest oder einen Arginylrest darstellen und mindestens einer der Reste X₁ oder X₂ oder X₃ ein Citrullinrest ist;
b) einem Peptid, das durch die Sequenz GPX₁VVEX₂HQSACKDS (SEQ ID NO:2) definiert ist, wobei X₁ und X₂ jeweils einen Citrullinrest oder einen Arginylrest darstellen und mindestens einer der Reste X₁ oder X₂ ein Citrullinrest ist;
c) einem Peptid, das durch die Sequenz SGIGTLDGFX₁HX₂HPD (SEQ ID NO:3) definiert ist, wobei X₁ und X₂ jeweils einen Citrullinrest oder einen Arginylrest darstellen und mindestens einer der Reste X₁ oder X₂ ein Citrullinrest ist;
d) einem Peptid, das durch die Sequenz VDIDIKIX₁SCX₂GSCS (SEQ ID NO:4) definiert ist, wobei X₁ und X₂ jeweils einen Citrullinrest oder einen Arginylrest darstellen und mindestens einer der Reste X₁ oder X₂ ein Citrullinrest ist;
e) einem Peptid, das durch die Sequenz X₁GHAKSX₂PVX₃GIHTS (SEQ ID NO:12) definiert ist, wobei X₁, X₂ und X₃ jeweils einen Citrullinrest oder einen Arginylrest darstellen und mindestens einer der Reste X₁ oder X₂ oder X₃ ein Citrullinrest ist;
f) einem Peptid, das mindestens 5 aufeinanderfolgende Aminosäuren, darunter mindestens einen Citrullinrest, aus einem der oben genannten Peptide a) bis e) umfasst.

5. Hybridmolekül nach einem der Ansprüche 1 bis 3, wobei das Peptid ausgewählt ist aus der Gruppe bestehend aus: SEQ ID NO:5, SEQ ID NO:6, SEQ ID NO:7, SEQ ID NO:8, SEQ ID NO:9, SEQ ID NO:10, SEQ ID NO:11, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22 und SEQ ID NO:23, stärker bevorzugt ausgewählt aus der Gruppe bestehend aus: SEQ ID NO:5, SEQ ID NO:6, SEQ ID NO:14, SEQ ID NO:18 und SEQ ID NO:19.

6. Hybridmolekül nach einem der vorangehenden Ansprüche, wobei das Fc-Fragment ein humanes Fc-Fragment, insbesondere von IgG, stärker bevorzugt von IgG₁ ist.

7. Hybridmolekül nach einem der vorangehenden Ansprüche, wobei das Fc-Fragment vom Wildtyp oder mutiert ist, wobei das mutierte Fc-Fragment bevorzugt mindestens die folgenden Mutationen umfasst:
- L234A und L235A, oder
- L234A, L235A und P329G, oder
- G236A, S239D und I332E, oder
- G236A, S239D, A330L und I332E, oder
- S239D, H268F, S324T und I332E,
wobei die Nummerierung in der Sequenz eines humanen IgG₁ gemäß EU-Nummerierung angegeben ist.

8. Hybridmolekül nach einem der vorangehenden Ansprüche, wobei
- das Fc-Fragment an mindestens ein Azid gebunden ist und das Peptid an ein Alkin, wie beispielsweise ein Cyclooctin, und insbesondere an DBCO, gebunden ist, oder
- das Fc-Fragment an mindestens ein Alkin, wie beispielsweise Cyclooctyn, und insbesondere DBCO, gebunden ist und das Peptid an ein Azid gebunden ist, oder
- das Fc-Fragment an mindestens ein Azid gebunden ist, und das Peptid an einen Spacer gebunden ist, der seinerseits an ein Alkin, wie beispielsweise Cyclooctyn, und insbesondere an DBCO, gebunden ist, oder
- das Fc-Fragment an mindestens ein Alkin, wie beispielsweise Cyclooctyn, und insbesondere an DBCO, gebunden ist und das Peptid an einen Spacer gebunden ist, der seinerseits an ein Azid gebunden ist, oder
- das Fc-Fragment an mindestens einen Spacer gebunden ist, der seinerseits an ein Azid gebunden ist, und das Peptid an ein Alkin, wie beispielsweise Cyclooctyn, und insbesondere an DBCO, gebunden ist, oder
- das Fc-Fragment an mindestens einen Spacer gebunden ist, der seinerseits an ein Alkin, wie beispielsweise Cyclooctyn, und insbesondere an DBCO, gebunden ist, und das Peptid an ein Azid gebunden ist, oder
- das Fc-Fragment an mindestens einen Spacer gebunden ist, der seinerseits an ein Azid gebunden ist, und das Peptid an einen Spacer gebunden ist, der seinerseits an ein Alkin, wie beispielsweise Cyclooctyn, und insbesondere an DBCO, gebunden ist, oder
- das Fc-Fragment an mindestens einen Spacer gebunden ist, der seinerseits an ein Alkin, wie beispielsweise Cyclooctyn, und insbesondere an DBCO, gebunden ist, und das Peptid an einen Spacer gebunden ist, der seinerseits an ein Azid gebunden ist,
wobei die kovalente Bindung zwischen dem Fc-Fragment und dem Peptid, gegebenenfalls in Gegenwart eines oder mehrerer Spacer, zwischen dem Azid und dem Alkin hergestellt wird.

9. Hybridmolekül nach einem der vorangehenden Ansprüche, wobei das Molekül dargestellt ist durch:
- ein Fc-Fragment vom Wildtyp, das an mindestens einen PEGn-Spacer gebunden ist, der seinerseits an ein Azid gebunden ist, das kovalent an ein Cyclooctyn gebunden ist, das an ein durch SEQ ID NO:19 oder SEQ ID NO:18 dargestelltes Peptid gebunden ist,
- ein die Mutationen S239D, H268F, S324T und I332E umfassendes Fc-Fragment, das an mindestens einen PEGn-Spacer gebunden ist, der seinerseits an ein Azid gebunden ist, das kovalent an ein Cyclooctyn gebunden ist, das an ein durch SEQ ID NO:19 oder SEQ ID NO:18 dargestelltes Peptid gebunden ist,
- ein die Mutationen L234A, L235A und P329G umfassendes Fc-Fragment, das an mindestens einen PEGn-Spacer gebunden ist, der seinerseits an ein Azid gebunden ist, das kovalent an ein Cyclooctyn gebunden ist, das an ein durch SEQ ID NO:19 oder SEQ ID NO:18 dargestelltes Peptid gebunden ist,
- ein Fc-Fragment vom Wildtyp, das an einen PEGn-Spacer gebunden ist, der seinerseits an mindestens ein Azid gebunden ist, das kovalent an ein Cyclooctyn gebunden ist, das an einen PEGn-Spacer gebunden ist, der seinerseits an ein durch SEQ ID NO:19 oder SEQ ID NO:18 dargestelltes Peptid gebunden ist,
- ein die Mutationen S239D, H268F, S324T und I332E umfassendes FC-Fragment, das an mindestens einen PEGn-Spacer gebunden ist, der seinerseits an ein Azid gebunden ist, das kovalent an ein Cyclooctyn gebunden ist, das an einen PEGn-Spacer gebunden ist, der seinerseits an ein durch SEQ ID NO:19 oder SEQ ID NO:18 dargestelltes Peptid gebunden ist,
wobei n bevorzugt eine ganze Zahl zwischen 1 und 10, stärker bevorzugt 1, 2 , 3 oder 4 ist.

10. Hybridmolekül nach einem der vorangehenden Ansprüche zur Verwendung als Medikament, insbesondere zur Verwendung bei der Behandlung von Autoimmunerkrankungen, die mit der Produktion von Anti-citrulliniertes Protein-Auto-Antikörpern im Zusammenhang stehen, insbesondere des Gougerot-Sjögren-Syndroms und rheumatoider Arthritis.

11. Hybridmolekül nach einem der vorangehenden Ansprüche zur Verwendung als Medikament in Kombination mit einem zweiten Hybridmolekül, wobei das zweite Hybridmolekül mindestens ein von Fibrin abgeleitetes Peptid mit mindestens einem Citrullinrest umfasst, wobei das Peptid kovalent an mindestens einen Antikörper oder an mindestens ein Antikörperfragment gebunden ist, wobei der Antikörper oder das Fragment in der Lage ist, an CD38 und/oder CD138 zu binden, wobei gegebenenfalls zwischen dem Peptid und dem Antikörper oder dem Fragment ein oder mehrere Spacer vorliegen.

## Claims

1. A hybrid molecule comprising at least one antibody Fc fragment covalently linked to at least one fibrin-derived peptide having at least one citrullyl residue, at least one spacer being optionally present between said Fc fragment and said peptide, said peptide being recognized by the anti-citrullinated protein autoantibodies and being derived from all or part of the sequence of the α or β chain of a vertebrate fibrin by substitution of at least one arginyl residue by a citrullyl residue.

2. A hybrid molecule according the preceding claim, said vertebrate fibrin being a mammalian fibrin, more particularly a human fibrin.

3. A hybrid molecule according to any of the preceding claims, wherein said spacer is a polymer containing one or more repeating units containing the ether group, said spacer preferably being polyethylene glycol of formula PEGn, wherein n represents an integer between 1 and 100, preferably between 1 and 10 and in particular 1, 2, 3, 4 or 8.

4. A hybrid molecule according to any one of the preceding claims, wherein said peptide comprises at least one citrullyl residue, and is selected from the group consisting of:
a) a peptide defined by the sequence X₁PAPPPISGGGYX₂AX₃ (SEQ ID NO: 1) wherein X₁, X₂, and X₃ each represent a citrullyl residue or an arginyl residue, and at least one of the X₁ or X₂ or X₃ residues is a citrullyl residue;
b) a peptide defined by the sequence GPX₁VVEX₂HQSACKDS (SEQ ID NO: 2) wherein X₁ and X₂ each represent a citrullyl residue or an arginyl residue, and at least one of the X₁ or X₂ residues is a citrullyl residue;
c) a peptide defined by the sequence SGIGTLDGFX₁HX₂HPD (SEQ ID NO: 3) wherein X₁ and X₂ each represents a citrullyl residue or an arginyl residue, and at least one of the X₁ or X₂ residues is a citrullyl residue;
d) a peptide defined by the sequence VDIDIKIX₁SCX₂GSCS (SEQ ID NO: 4) wherein X₁ and X₂ each represent a citrullyl residue or an arginyl residue, and at least one of the X₁ or X₂ residues is a citrullyl residue;
e) a peptide defined by the sequence X₁GHAKSX₂PVX₃GIHTS (SEQ ID NO: 12) wherein X₁, X₂ and X₃ each represent a citrullyl residue or an arginyl residue, and at least one of the X₁ or X₂ or X₃ residues is a citrullyl residue;
f) a peptide comprising at least 5 consecutive amino acids, including at least one citrullyl residue, from one of the peptides a) to e) above.

5. A hybrid molecule according to any one of claims 1-3, wherein said peptide is selected from the group consisting of: SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 and SEQ ID NO: 23, more particularly selected from the group consisting of: SEQ ID NO: 5, SEQ ID NO: 6, SEQ ID NO: 14, SEQ ID NO: 18 and SEQ ID NO: 19.

6. A hybrid molecule according to any one of the preceding claims, wherein the said Fc fragment is a human Fc fragment, in particular of IgG, more particularly of IgG1.

7. A hybrid molecule according to any of the preceding claims, wherein said Fc fragment is wild-type or mutated, said mutated Fc fragment preferably comprising at least the following mutations:
- L234A and L235A, or
- L234A, L235A and P329G, or
- G236A, S239D and I332E, or
- G236A, S239D, A330L and I332E, or
- S239D, H268F, S324T and I332E,
the numbering being indicated in the sequence of a human IgG1 according to the EU index.

8. A hybrid molecule according to any one of the preceding claims, wherein:
- the Fc fragment is coupled to at least one azide and said peptide is coupled to an alkyne, such as a cyclooctyne, and in particular DBCO, or
- the Fc fragment is coupled to at least one alkyne, such as a cyclooctyne, and in particular DBCO, and said peptide is coupled to an azide, or
- the Fc fragment is coupled to at least one azide and said peptide is bound to a spacer, itself coupled to an alkyne, such as a cyclooctyne, and in particular DBCO, or
- the Fc fragment is coupled to at least one alkyne, such as a cyclooctyne, and in particular DBCO, and said peptide is bound to a spacer, itself coupled to an azide, or
- the Fc fragment is bound to at least one spacer, itself coupled to an azide, and said peptide is coupled to an alkyne, such as a cyclooctyne, and in particular DBCO, or
- the Fc fragment is bound to at least one spacer, itself coupled to an alkyne, such as a cyclooctyne, and in particular DBCO, and said peptide is coupled to an azide, or
- the Fc fragment is bound to at least one spacer, itself coupled to an azide, and said peptide is bound to a spacer, itself coupled to an alkyne, such as a cyclooctyne, and in particular DBCO, or
- the Fc fragment is bound to at least one spacer, itself coupled to an alkyne, such as a cyclooctyne, and in particular DBCO, and said peptide is bound to a spacer, itself coupled to an azide,
the covalent bond between said Fc fragment and said peptide, optionally in the presence of one or more spacers, being created between the azide and the alkyne.

9. A hybrid molecule according to any one of the preceding claims, said molecule being represented by:
- a wild-type Fc fragment that is bound to at least one PEGn spacer that is itself coupled to an azide covalently bound to a cyclooctyne that is coupled to a peptide represented by SEQ ID NO: 19 or SEQ ID NO: 18,
- an Fc fragment comprising the S239D, H268F, S324T and I332E mutations that is bound to at least one PEGn spacer that is itself coupled to an azide covalently bound to a cyclooctyne that is coupled to a peptide represented by SEQ ID NO: 19 or SEQ ID NO: 18,
- an Fc fragment comprising the L234A, L235A and P329G mutations that is bound to at least one PEGn spacer that is itself coupled to an azide covalently bound to a cyclooctyne that is coupled to a peptide represented by SEQ ID NO: 19 or SEQ ID NO: 18,
- a wild-type Fc fragment that is bound to a PEGn spacer that is itself coupled to at least one azide covalently bound to a cyclooctyne coupled to a PEGn spacer that is itself bound to a peptide represented by SEQ ID NO: 19 or SEQ ID NO: 18,
- an Fc fragment comprising the S239D, H268F, S324T and I332E mutations that is bound to at least one PEGn spacer that is itself coupled to an azide covalently bound to a cyclooctyne coupled to a PEGn spacer that is itself bound to a peptide represented by SEQ ID NO: 19 or SEQ ID NO: 18,
n preferably representing an integer between 1 and 10, more particularly 1, 2, 3 or 4.

10. A hybrid molecule according to any one of the preceding claims for use as a medicinal product, in particular for use in the treatment of autoimmune diseases associated with the production of anti-citrullinated protein autoantibodies, in particular Gougerot-Sjögren syndrome and rheumatoid arthritis.

11. A hybrid molecule according to any one of the preceding claims for use as a medicinal product in combination with a second hybrid molecule, said second hybrid molecule comprising at least one fibrin-derived peptide having at least one citrullyl residue, said peptide being covalently bound to at least one antibody or to at least one antibody fragment, said antibody or fragment being capable of binding to CD38 and/or CD138, one or more spacers being optionally present between said peptide and said antibody or said fragment.
